# EUROPEAN PATENT APPLICATION

(11) **EP 1 371 376 A1**
(43) Date of publication of application: **17.12.2003**
(21) Application number: 02705294.3
(22) Date of filing: 18.03.2002
(51) Int. Cl.: A61K 45/00, A61K 31/517, A61P 17/04, C07D 239/94, C07D 239/96, C07D 401/06

(54) **ANTIPRURITICS**

(30) Priority: 19.03.2001 JP 2001078933
(71) Applicant: Nippon Shinyaku Co., Ltd., Kyoto-shi Kyoto 601-8550 (JP)
(72) Inventor: OYAMA, Tatsuya, Nagaokakyo-shi, Kyoto 617-0828 (JP); SAKANO, Kyoko, Kyoto-shi, Kyoto 600-8894 (JP)
(74) Representative: VOSSIUS & PARTNER
(86) International application number: JP0202546
(87) International publication number: WO02074341

(57) **Abstract**

The present invention relates to an antipruritic agent comprising a nociceptin antagonist as an active ingredient. The nociceptin antagonist can be used as a preventive or remedy for diseases associated with itching (for example, atopic dermatitis and urticaria), local pruritus cutaneous caused by insect excretion and secretion, nodular prurigo, kidney dialysis, diabetes, blood disease, liver disease, kidney disease, incretion and metabolic disorder, viscera malignant tumor, hyperthyroidism, autoimmune disease, multiple sclerosis, neurologic disease, psychoneurosis, allergic conjunctivitis, spring catarrh, atopic keratoconjunctivitis, or itching caused by excess use of laxuries and drugs because it has excellent scrtaching behavior suppressing effect, that is, antiitching effect and antipruritic effect.

## Description

### TECHNICAL FIELD

The present invention relates to an antipruritic agent.

### BACKGRUND ART

Itching is a sensation (pruritic sensation) which takes place at the surface of the skin and the mucosa adjacent to the skin. The pruritic sensation is a sensation which senses a parasite and an irritant of the skin surface and removes an invading substance and an irritant by scratching. Itching is a sensation which can be easily understood as a sensation causing an impulse to scratch, but its mechanism has not been elucidated completely.

Disorder characterized by pruritus is separated two types: iching skin disorder (for example, atopic dermatitis, urticaria, psoriasis, xeroderma and trichophytia) and pruritus cutaneous which is not associated with skin disorder and provokes itching due to kidney dialysis and internal organ diseases [for example, diabetes, blood disease, cholestatic hepatitis (primary biliary liver cirrhosis) and kidney disease], hyperthyroidism and multiple sclerosis. In addition, the disease associated with severe itching includes diseases of cornea and conjunctiva, for example, allergic conjunctivitis. Recently, patients with these diseases have repidly increased to constitute a large problem in view of QOL (quality of life). Most itching diseases are common in the fact that vicious circle is caused by injure due to scratching. Histamine is known as a typical itching-producing substance and provokes itching in case it is externally added and is internally isolated from mast cells.

An antihistaminic agent, an antiallergic agent and a steroid external agent are used for the treatment of pruritic dermatitis. However, because of its side effects, all of them are not satisfied for the treatment of itching due to pruritic dermatitis because side effects arise. And it has recently been reported that there are compounds other than histamine take part in itching due to atopic dermatitis. In many clinical cases, the antihistaminic agent and the antiallergic agent do not actually exert a remarkable effect on itching due to atopic dermatitis. In the treatment of pruritus cutaneous, the antihistaminic agent or the steroid external agent is prescribed sometimes, however, they exert almost no effect, and thus an effective therapy does not exist at present. As described above, there are no satisfactory medicaments for diseases associated with itching and it is required to develop a medicament which effectively suppresses itching regardless of causative diseases from a clinical point of view.

At present, some nociceptin antagonists have been developed as an analgesic. It is known that the compounds of the general formulas (I), (III) and (IV) of claim 2 have a nociceptin antagonism and are useful as the analgesic (International Publications WO 01/72710, WO 98/54168 and WO 99/48492). However, it is not known at all that these compounds have an antipruritic effect.

### DISCLOSURE OF THE INVENTION

An object of the present invention is to provide an excellent antipruritic agent having a novel action mechanism and, more particularly, to provide an antipruritic agent having a nociceptin antagonism as the action mechanism.

The present inventors have intensively studied about a compound having an action mechanism which suppresses a transmission path of a pruritic sensation. As a result, they have found that a nociceptin antagonist has an antipruritic effect. More specifically, they have found that the compound of the general formula (II) of claim 2 has a nociceptin antagonism and also has an antipruritic effect, and that the compounds of the general formulas (I), (III) and (IV) of claim 2 known to have a nociceptin antagonism have an antipruritic effect. Thus, the present invention has been completed.
The present invention provides:
(1) An antipruritic agent comprising a nociceptin antagonist as an active ingredient;
(2) The antipruritic agent according to claim 1, wherein the nociceptin antagonist is a compound which is represented by any one of the following general formulas (I) to (IV), or a salt thereof:

### 1) the general formula (I):

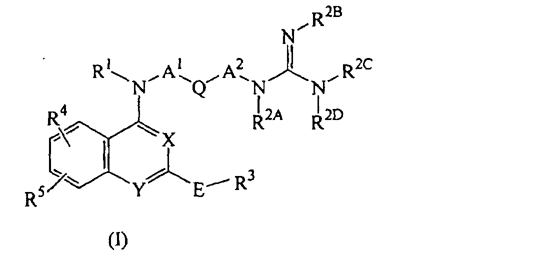

wherein X and Y are the same or different and represent a nitrogen atom or CH;
R¹ represents a hydrogen atom or alkyl;
A¹ and A² are the same or different and represent, (1) a single bond, or (2) a divalent aliphatic hydrocarbon group which may be substituted and may have 1 to 3 unsaturated bonds at any position (the aliphatic hydrocarbon group may contain one hetero atom selected from the group consisting of -NH-, O and S, and may include 1 to 3 unsaturated bonds at any position);
Q represents (1) a single bond, (2) an optionally substituted 3- to 8-membered cycloalkylene group, (3) an optionally substituted phenylene group, or (4) an optionally substituted 4-to 8-membered divalent heterocyclic group;
R^{2A}, R^{2C} and R^{2D} are the same or different and represent a hydrogen atom, alkyl or phenyl, and R^{2B} represents a hydrogen atom, alkyl, a cyano group, a nitro group or phenyl, or two nitrogen atoms of a guanidino group are cyclized together with one or two substituents among R^{2B}, R^{2C} and R^{2D} to form a saturated or unsaturated 5- or 6-membered ring;
or are taken together as -N(R¹)-A¹-Q-A²-N(R^{2A})- to form a 5-to 7-membered ring;
E represents (1) an ethenylene group, (2) -NRCO-, (3)-NRCONH-, (4) -CONR-, (5) an ethynylene group, (6) -NRSO₂-, or (7) an aminoalkylene group (wherein R represents hydrogen or an optionally substituted alkyl);
R³ represents an optionally substituted phenyl group or an optionally substituted heterocyclic group;
R⁴ and R⁵ are the same or different and represent (1) a hydrogen atom, alkyl, alkoxy, aralkyloxy, halogen, nitro, hydroxy, alkoxycarbonyl, -NR⁶R⁷, -NR⁶COR⁷, -NR⁶SO₂R⁷, or-CONR⁶R⁷ (wherein R⁶, R⁷ are the same or different and represent a hydrogen atom or alkyl), or (2) adjacent R⁴ and R⁵ may be combined to form -O(CH₂)ₙO- (wherein n represents an integer of 1 or 2) or -CH=CH-CH=CH-;

### 2) the general formula (II):

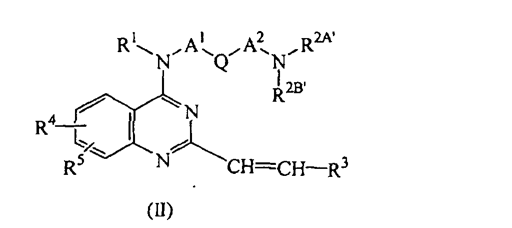

wherein R¹ represents a hydrogen atom or alkyl;
A¹ and A² are the same or different and represent (1) a single bond, or (2) a divalent aliphatic hydrocarbon group which may be substituted and may have 1 to 3 unsaturated bonds at any position (the aliphatic hydrocarbon group may have one hetero atom selected from the group consisting of -NH-, O and S, and may include 1 to 3 unsaturated bonds at any position);
Q represents (1) a single bond, (2) an optionally substituted 3- to 8-membered cycloalkylene group, (3) an optionally substituted phenylene group, or (4) an optionally substituted 4-to 8-membered divalent heterocyclic group;
R^{2A'} and R^{2B'} are the same or different and represent a hydrogen atom or alkyl;
or are taken together as -N(R¹)-A¹-Q-A²-N(R^{2A'})- to form a 5-to 7-membered ring;
R³ represents an optionally substituted phenyl group or an optionally substituted heterocyclic group;
R⁴ and R⁵ are the same or different and represent, (1) a hydrogen atom, alkyl, alkoxy, aralkyloxy, halogen, nitro, hydroxy, alkoxycarbonyl, -NR⁶R⁷, -NR⁶COR⁷, -NR⁶SO₂R⁷, or-CONR⁶R⁷ (wherein R⁶ and R⁷ are the same or different and represent a hydrogen atom or alkyl), or (2) adjacent R⁴ and R⁵ may be combined to form -O(CH₂)ₙO- (wherein n represents an integer of 1 or 2) or -CH=CH-CH=CH-);

### 2) a compound represented by the general formula (III):

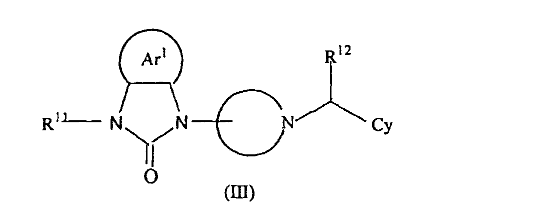

a compound which is represented as followed, or a salt thereof or a ester thereof; 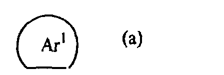 [wherein represents an aromatic carbon ring or an aromatic heterocycle, which may have a substituent selected from the group consisting of halogen atom, lower alkyl group, amino group, lower alkylamino group, di-lower alkylamino group, hydroxyl group, lower alkoxy group and carboxyl group; Cy represents a C3-20 mono-, di- or tricyclic aliphatic carbon ring group which may have a substituent selected from the group consisting of halogen atom, lower alkylidene group, lower alkenyl group, lower alkynyl group, amino group, lower alkylamino group, di-lower alkylamino group, lower alkoxy group and group represented by -R¹⁴; 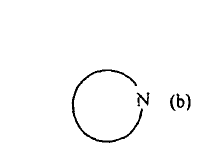 represents a C3-14 mono- or dicyclic aliphatic nitrogen-containing heterocyclic group which may have a substituent selected from the group consisting of halogen atom, lower alkylidene group, lower alkenyl group, lower alkynyl group, amino group, lower alkylamino group, di-lower alkylamino group, hydroxyl group, lower alkoxy group, carboxyl group, lower alkoxycarbonyl group, carbamoyl group, lower alkylcarbamoyl group, di-lower alkylcarbamoyl group and group represented by - R¹³; R¹¹ represents a hydrogen atom, a lower alkenyl group, a lower alkynyl group, a lower cycloalkyl group, an amino group, a lower alkylamino group, a di-lower alkylamino group, a hydroxyl group, a lower alkoxy group, a carboxyl group, a lower alkoxycarbonyl group, a carbamoyl group, a lower alkylcarbamoyl group or a di-lower alkylcarbamoyl group, or a lower alkyl group which may have a substituent selected from the group consiting of halogen atom, lower cycloalkyl group, amino group, lower alkylamino group, di-lower alkylamino group, lower alkylsulfonylamino group, aminosulfonylamino group, (lower alkylamino)sulfonylamino group, (di-lower alkylamino)sulfonylamino group, carbamoyl amino group, (lower alkylcarbamoyl)amino group, (di-lower alkylcarbamoyl)amino group, hydroxyl group, lower alkoxy group, carbamoyloxy group, lower alkylcarbamoyloxy group, di-lower alkylcarbamoyloxy group, carboxyl group, lower alkoxycarbonyl group, carbamoyl group, lower alkylcarbamoyl group, di-lower alkylcarbamoyl group and group represented by -Ar²; R¹² represents a hydrogen atom or a lower alkyl group; R¹³ represents a lower alkyl group which may have a substituent selected from the group consisting of amino group, lower alkylsulfonylamino group, aminosulfonylamino group, (lower alkylamino)sulfonylamino group, (di-lower alkylamino)sulfonylamino group, carbamoyl amino group, (lower alkylcarbamoyl)amino group, (di-lower alkylcarbamoyl)amino group, hydroxyl group, carbamoyloxy group, lower alkylcarbamoyloxy group, di-lower alkylcarbamoyloxy group, carboxyl group, lower alkoxycarbonyl group, carbamoyl group, lower alkylcarbamoyl group, di-lower alkylcarbamoyl group, aromatic heterocycle and group represented by -R¹⁵; R¹⁴ represents a lower alkyl group which may have a substituent selected from the group consisting of C3-10 cycloalkyl group, aromatic carbon ring group and aromatic heterocyclic group; R¹⁵ represents a lower alkylamino group, a di-lower alkylamino group or a lower alkoxy group, which may have an aromatic carbon ring group or an aromatic heterocyclic group; and Ar² represents an aromatic carbon ring group or an aromatic heterocyclic group, which may have a substituent selected from the group consisting of halogen atom, lower alkyl group, amino group, lower alkylamino group, di-lower alkylamino group, hydroxyl group, lower alkoxy group and carboxyl group]; and

### 4) a compound represented by the general formula (IV):

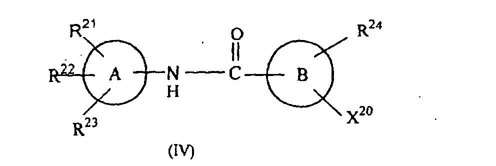

an amide derivative which is represented as followed, or a pharmaceutically acceptable salt thereof;
[wherein R²¹ and R²² are the same or different and each represents a hydrogen atom, a lower alkyl group which may be substituted with a hydroxyl group, an amino group, a lower alkylamino group or a di-lower alkylamino group; R²³ and R²⁴ are the same or different and each represents a hydrogen atom, a halogen atom or a lower alkyl group, the ring A represents an aryl group or a heterocyclic group, the ring B represents a phenyl group, a thienyl group, a furyl group, a pyrrolyl group, apyrrolidinyl group, an oxazolyl group or a cyclohexenyl group, X²⁰ represents a hydrogen atom, a halogen atom, a lower alkyl group which may be substituted with a lower alkoxy group, a lower alkenyl group, an amino group, a cyano group, or 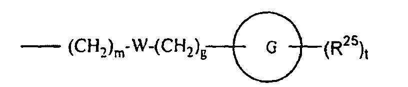 {wherein W represents a single bond, -CH=CR²⁶- (wherein R²⁶ represents a hydrogen atom or an aryl group), -0-, -S-, -NR²⁷- (wherein R²⁷ represents a hydrogen atom, a lower alkyl group or a lower alkoxycarbonyl group), a carbonyl group, a sulfinyl group or -NHCO-, the ring G represents an aryl group, a heterocyclic group, a cycloalkyl group or a fused aryl group, R²⁵ represents a halogen atom, a hydroxyl group, a lower alkoxy group which may be substitiuted with a lower alkoxy group, a lower alkyl group which may be substituted with any of a halogen atom, a hydroxyl group and a lower alkanoyloxy group, a lower alkoxy group which may be substituted with a lower alkoxy group, an amino group, a lower alkylamino group, a di-lower alkylamino group, a nitro group, a cyano group, a lower alkanoyl group, a lower alkanoyloxy group, a carboxy group, a lower alkoxycarbonyl group, a lower alkylsulfonyl group or a phenyl group, t is an integer of 0 or 1 to 5, which represents the number of substituents on the ring G, and when t is an integer of 2 to 5, R²⁵ may be the same or different, m represents an integer of 0 or 1 to 8, and g represents an integer of 0 or 1 to 4.}]
(3) The antipruritic agent, wherein the nociceptin antagonist is a compound represented by the above-mentioned formula (I), (III) or (IV);
(4) The antipruritic agent, wherein the nociceptin antagonist is a compound selected from the group consisting of (1R,2S)-N-amidino-2-{[2-(4-chlorobenzoylamino)-6-methoxyquinazolin-4-yl]amino}cyclohexylamine dihydrochloride, (1R,2S)-N-amidino-2-{[2-(4-chlorobenzoylamino)-6-methylquinazolin-4-yl]amino}cyctohexylamine dihydrochloride, 1-[(3R,4R)-1-cyclooctylmethyl-3-hydroxymethyl-4-piperidyl]-3-ethyl-1,3-dihydro-2H-benzimidazol-2-one hydrochloride and N-(4-amino-2-methyl-6-quinolyl)-2-[(4-ethylphenoxy)methyl]benzamide hydrochloride; and
(5) An antipruritic agent comprising a therapeutically active amount of a nociceptin antagonist and a pharmaceutically acceptable carrier or excipient.

A feature of the present invention lies in that an antipruritic effect, which has never been known in the prior art, has found in a nociceptin antagonist.

The present invention will be described in detail hereinafter.

Terms used in the present invention and definitions of substituents are as follows.

The term "nociceptin antagonist" as used herein refers to a drug which is combined with nociceptin receptor (also referred to as orphanin FQ) as a biologically active substance and exhibits an antagonism. The compound used as a nociceptin antagonist may be any compound as far as it has a nociceptin antagonism. For example, compounds represented by the above-mentioned formulas (I) to (IV) can be listed. Preferably, examples of the compound represented by the formula (II) include compounds described in the Examples, and examples of the compounds represented by the formulas (I), (III) and (IV) include compounds described hereinafter.

The term "antipruritic agent" as used herein refers to a drug for the suppression of itching due to atopic dermatitis, urticaria, psoriasis, xeroderma, trichophytia, vitiligo vulgaris, local pruritus cutaneous caused by insect excretion and secretion, nodular prurigo, kidney dialysis, diabetes, blood disease, liver disease, kidney disease, incretion and metabolic disorder, viscera malignant tumor, hyperthyroidism, autoimmune disease, multiple sclerosis, neurologic disease, psychoneurosis, allergic conjunctivitis, spring catarrh, atopic keratoconjunctivitis, or itching caused by excess use of laxuries and drugs.

Definitions of the respective substituents the general formulas (I) and (II) described in claim 2 of the present specification are as follows.

Examples of "alkyl" include a straight or branched alkyl having 1 to 6 carbon atoms, for example, methyl, ethyl, n-propyl, isopropyl, n-butyl, isobutyl, sec-butyl, tert-butyl, n-pentyl, 5-isopentyl, n-hexyl, and isohexyl. Particularly, alkyl having 1 to 4 carbon atoms is preferable.

Examples of "alkoxy" include a straight or branched alkoxy having 1 to 6 carbon atoms, for example, methoxy, ethoxy, n-propoxy, isopropoxy, n-butoxy, isobutoxy, sec-butoxy, tert-butoxy, n-pentyloxy, isopentyloxy, n-hexyloxy, and isohexyloxy. Particularly, alkoxy having 1 to 4 carbon atoms is preferable.

Examples of "aralkyloxy" include an aralkyloxy having 7 to 10 carbon atoms, for example, benzyloxy and phenethyloxy. Particularly, benzyloxy is preferable.

Examples of "divalent aliphatic hydrocarbon group" include a straight or branched alkylene having 1 to 6 carbon atoms (for example, methylene, ethylene, trimethylene, tetramethylene, pentamethylene, hexamethylene, 2-(ethyl)trimethylene, and 1-(methyl)tetramethylene), a straight or branched alkenylene having 2 to 6 carbon atoms (for example, vinylene and propenylene), and a straight or branched alkynylene having 2 to 6 carbon atoms (for example, ethynylene). Such an aliphatic hydrocarbon groups may have one hetero atom selected from the group consisting of NH, oxygen atom and sulfur atom.

"Cycloalkylene" may have an unsaturated bond and examples thereof include cycloalkylene having 3 to 8 carbon atoms, for example, cyclopropylene, cyclobutylene, cyclopentylene, cyclohexylene, cycloheptylene, cyclooctylene, cyclohexenylene, cycloheptenylene and cyclooctenylene. Such a cycloalkylene may have 1 to 2 substituents, and examples of such substituents may include alkyl, alkoxycarbonyl, carbamoyl, monoalkylcarbamoyl, dialkylcarbamoyl or alkoxy.

Examples of "halogen" include fluorine, chlorine, bromine and iodine atoms.

Examples of a heterocyclic ring in a "heterocyclic group" and "divalent heterocyclic group" may include a 4-to 8-membered monocyclic or fused ring which has 1 to 2 hetero atoms selected from the group consisting of nitrogen atom, oxygen atom and sulfur atom, and which may have 1 to 4 unsaturated bonds. Examples of R³ include 2-pyridyl, 3-pyridyl, 4-pyridyl, 2-pyrimidinyl, 4-pyrimidinyl, 5-pyrimidinyl, 3-pyridazinyl, 4-pyridazinolyl, 2-pyrazinyl and 3-pyrazinyl. Such a heterocyclic group may have 1 to 2 substituents, and examples of the substituents include alkyl, alkoxy, alkoxycarbonyl, carbamoyl, monoalkylcarbamoyl, dialkylcarbamoyl, sulfamoyl, monoalkylsulfamoyl, dialkylsulfamoyl, alkylsulfonylamino, N-(alkyl)alkylsulfonylamino, amino, monoalkylamino, dialkylamino, nitro, halogen, cyano, hydroxy or trifluoromethyl. Examples of a heterocyclic ring in a heterocyclic group Q may include pyridine, pyrimidine, piperazine, homopiperazine, furan and thiophene. The heterocyclic group Q may have 1 to 2 substituents, and examples of such substituents include alkyl, alkoxy, alkoxycarbonyl, carbamoyl, monoalkylcarbamoyl, dialkylcarbamoyl, amino, monoalkylamino or dialkylamino.

A "phenylene group" may have 1 to 2 substituents, and examples of such substituents include alkyl, alkoxy, alkoxycarbonyl, carbamoyl, monoalkylcarbamoyl, dialkylcarbamoyl, sulfamoyl, monoalkylsulfamoyl, dialkylsulfamoyl, amino, monoalkylamino, dialkylamino, hydroxy, nitro, halogen, cyano and trifluoromethyl.

Examples of a ring represented by -N(R¹)-A¹-Q-A²-N(R^{2A}) include a 5- to 7-membered saturated ring, such as piperazino or homopiperazino.

Examples of "salt" include pharmacologically acceptable salts, for example, salts of inorganic acids such as hydrochloric acid, sulfuric acid, nitric acid, phosphoric acid, hydrofluoric acid and hydrobromic acid, or salts of organic acids such as acetic acid, tartaric acid, lactic acid, citric acid, fumaric acid, maleic acid, succinic acid, methanesulfonic acid, ethanesulfonic acid, benzenesulfonic acid, toluenesulfonic acid, naphthalenesulfonic acid and camphorsulfonic acid.

Preferred is a compound of the general formula (I) wherein X and Y represent a nitrogen atom, R¹ represents a hydrogen atom or alkyl, A¹ and A² are the same or different and represent (1) a single bond or (2) an optionally substituted alkylene, Q represents (1) a single bond, (2) an optionally substituted 4- to 8-membered cycloalkylene group, (3) an optionally substituted phenylene group, or (4) an optionally substituted 5- to 7-membered divalent heterocyclic group, R^{2A}, R^{2B}, R^{2C} and R^{2D} are the same or different and represent a hydrogen atom, alkyl or phenyl, or are taken together as -N(R¹)-A¹-Q-A²-N(R^{2A})- to form a 5- to 7-membered ring, E represents (1) ethenylene, (2)-NRCO-, or (3)-CONR-, and R⁴ and R⁵ (1) are the same or different and represent a hydrogen atom, alkyl, alkoxy, aralkyloxy, halogen, nitro, hydroxy, or alkoxycarbonyl, or (2) adjacent R⁴ and R⁵ are combined to form -O(CH₂)ₙO- (wherein n represents an integer of 1 or 2) or -CH=CH-CH=CH-. More preferred is a compound wherein X and Y represent a nitrogen atom, R' represents a hydrogen atom, A¹ and A² are the same or different and represent (1) a single bond or (2) an optionally substituted alkylene, Q represents (1) a single bond, (2) an optionally substituted 5- to 7-membered cycloalkylene group, or (3) an optionally substituted phenylene group, R^{2A}, R^{2B}, R^{2C} and R^{2D} are the same or different and represent a hydrogen atom, alkyl or phenyl, E represents (1) ethenylene or (2) -NRCO-, and R⁴ and R⁵ are the same or different and represent a hydrogen atom, alkyl, alkoxy, aralkyloxy, halogen or nitro.

Preferred is a compound of the general formula (II) wherein R' represents a hydrogen atom or alkyl, A¹ and A² are the same or different and represent (1) a single bond or (2) an optionally substituted alkylene, Q represents (1) a single bond, (2) an optionally substituted 4- to 8-membered cycloalkylene group, (3) an optionally substituted phenylene group, or (4) an optionally substituted 5- to 7-membered divalent heterocyclic group, R^{2A'} and R^{2B'} are the same or different and represent a hydrogen atom or alkyl, or are taken together as -N(R¹)-A¹-Q-A²-N(R^{2A'})- to form a 5- to 7-membered ring, R⁴ and R⁵ (1) are the same or different and represent a hydrogen atom, alkyl, alkoxy, aralkyloxy, halogen, nitro, hydroxy, or alkoxycarbonyl, or (2) adjacent R⁴ and R⁵ are combined to form -O(CH₂)ₙO- (wherein n represents an integer of 1 or 2) or -CH=CH-CH=CH-. More preferred is a compound wherein R¹ represents a hydrogen atom, A¹ and A² are the same or different and represent (1) a single bond or (2) an optionally substituted alkylene, Q represents (1) a single bond, (2) an optionally substituted 5- to 7-membered cycloalkylene group, or (3) an optionally substituted phenylene group, R^{2A'} and R^{2B'} are the same or different and represent a hydrogen atom or alkyl, and R⁴ and R⁵ are the same or different and represent a hydrogen atom, alkyl, alkoxy, aralkyloxy, halogen, or nitro.

Definitions of the respective substituents of the general formula (III) described in claim 2 of the present specification are the same as in International Publication WO98/54168.

The term "halogen atom" as used herein means fluorine, chlorine, bromine and iodine atoms.

The term "lower alkyl group" as used herein means a straight or branched alkyl group having 1 to 6 carbon atoms and examples thereof include methyl group, ethyl group, propyl group, isopropyl group, butyl group, isobutyl group, sec-butyl group, tert-butyl group, pentyl group, isopentyl group, neopentyl group, tert-pentyl group, 1 -methylbutyl group, 2-methylbutyl group, 1,2-dimethylpropyl group, 1-ethylpropyl group, hexyl group, isohexyl group, 1-methylpentyl group, 2-methylpentyl group, 3-methylpentyl group, 1,1-dimethylbutyl group, 1,2-dimethylbutyl group, 2,2-dimethylbutyl group, 1-ethylbutyl group, 1,1,2-trimethylpropyl group, 1,2,2-trimethylpropyl group, 1-ethyl-2-methylpropyl group, and 1-ethyl-1-methylpropyl group.

The term "lower alkylamino group" as used herein means an amino group which is mono-substituted with the lower alkyl group, and examples thereof include methylamino group, ethylamino group, propylamino group, isopropylamino group, butylamino group, sec-butylamino group, and tert-butylamino group.

The term "di-lower alkylamino group" as used herein means an amino group which is di-substituted with the lower alkyl group, and examples thereof include dimethylamino group, diethylamino group, ethylmethylamino group, dipropylamino group, methylpropylamino group, and diisopropylamino group.

The term "lower alkoxy group" as used herein means an alkoxy group having the lower alkyl group, namely an alkoxy group having 1 to 6 carbon atoms, and examples thereof include methoxy group, ethoxy group, propoxy group, isopropoxy group, butoxy group, isobutoxy group, tert-butoxy group, and pentyloxy group.

The term "aromatic carbon ring" as used herein means a benzene ring, a naphthalene ring or an anthracene ring.

The term "aromatic heterocycle" as used herein means a 5- or 6-membered monocyclic aromatic heterocycle which has 1 or 2 or more, preferably 1 to 3 hetero atoms, which are the same or different and are selected from the group consisting of oxygen atom, nitrogen atom and sulfur atom, or a fused cyclic aromatic heterocycle obtained by fusing the monocyclic aromatic heterocycle with the aromatic carbon ring or fusing the same or different monocyclic aromatic heterocycles with each other, and examples thereof include pyrrole ring, furan ring, thiophene ring, imidazole ring, pyrazole ring, thiazole ring, isothiazole ring, oxazole ring, isoxazole ring, triazole ring, tetrazole ring, oxadiazole ring, thiadiazole ring, pyridine ring, pyrazine ring, pyrimidine ring, pyridazine ring, indole ring, benzofuran ring, benzothiophene ring, benzoimidazole ring, benzoxazole ring, benzoisoxazole ring, benzothiazole ring, benzoisothiazole ring, indazole ring, purine ring, quinoline ring, isoquinoline ring, phthalazine ring, naphthylidine ring, quinoxaline ring, quinazoline ring, cinnoline ring, and pteridine ring.

The term "aromatic carbon ring group" as used herein means a group formed from the aromatic carbon ring, and examples thereof include phenyl group, naphthyl group or anthryl group.

The term "aromatic heterocyclic group" as used herein means a group formed from the aromatic heterocycle, and examples thereof include pyrrolyl group, furyl group, thienyl group, imidazolyl group, pyrazolyl group, thiazolyl group, isothiazolyl group, oxazolyl group, isoxazolyl group, triazolyl group, tetrazolyl group, oxadiazolyl group, thiadiazolyl group, pyridyl group, pyrazinyl group, pyrimidinyl group, pyridazinyl group, indolyl group, benzofuranyl group, benzothienyl group, benzoimidazolyl group, benzoxazolyl group, benzisoxazolyl group, benzothiazolyl group, benzoisothiazlyl group, indazolyl group, purinyl group, quinolyl group, isoquinolyl group, phthalazinyl group, naphthylidinyl group, quinoxalinyl group, quinazolynyl group, cinnolinyl group, and pteridinyl group.

The term "lower alkylidene group" as used herein means a straight or branched alkylidene group having 1 to 6 carbon atoms, and examples thereof include methylene group, ethylidene group, propylidene group, isopropylidene group, and butylidene group.

The term "lower alkenyl group" as used herein means a straight or branched alkenyl group having 2 to 6 carbon atoms, and examples thereof include vinyl group, 1-propenyl group, 2-propenyl group, isopropenyl group, 3-butenyl group, 2-butenyl group, 1-butenyl group, 1-methyl-2-propenyl group, 1-methyl-1-propenyl group, 1-ethyl-1-ethenyl group, 2-methyl-2-propenyl group, 2-methyl-1-propenyl group, 3-methyl-2-butenyl group and 4-pentenyl group.

The term "lower alkynyl group" as used herein means a straight or branched alkynyl group having 2 to 6 carbon atoms, and examples thereof include ethynyl group, 2-propynyl group, 1-methyl-2-propynyl group, 2-butynyl group, 1-methyl-2-butynyl group, and 2-pentynyl group.

The term "lower cycloalkyl group" as used herein means a cycloalkyl group having 3 to 6 carbon atoms, and examples thereof include cyclopropyl group, cyclobutyl group, cyclopentyl group, and cyclohexyl group.

The term "mono-, di- or tricyclic aliphatic carbon ring group" as used herein means a saturated or unsaturated aliphatic carbon ring group which is a mono-, di- or tricyclic ring group, and examples thereof include cyclopropyl group, cyclobutyl group, cyclopentyl group, cyclohexyl group, cycloheptyl group, cyclooctyl group, cyclononyl group, cyclodecyl group, cycloundecyl group, cyclododecyl group, 1-cyclopentenyl group, 2-cyclopentenyl group, 3-cyclopentenyl group, 1-cyclohexenyl. group, 2-cyclohexenyl group, 1,3-cyclohexadienyl group, 1-cycloheptenyl group, 2-cycloheptenyl group, 1,3-cycloheptadienyl group, 1-cyclooctenyl group, 2-cyclooctenyl group, 3-cyclooctenyl group, 4-cyclooctenyl group, 1,3-cyclooctadienyl group, 1-cyclononenyl group, 2-cyclononenyl group, 3-cyclononenyl group, 4-cyclononenyl group, 1,3-cyclononadienyl group, 1-cyclodecenyl group, 2-cyclodecenyl group, 3-cyclodecenyl group, 4-cyclodecenyl group, 1,3-cyclodecadienyl group, 1-cycloundecenyl group, 2-cycloundecenyl group, 1,3-cycloundecadienyl group, 1-cyclododecenyl group, 2-cyclododecenyl group, 1,3-cyclododecadienyl group, bicyclo[3.2.1]oct-1-yl group, bicyclo[3.2.1]oct-2-yl group, bicyclo[3.2.1]oct-3-yl group, bicyclo[3.2.1]oct-6-yl group, bicyclo[3.2.1]oct-8-yl group, bicyclo[4.4.0]dec-1-yl group, bicyclo[4.4.0]dec-2-yl group, bicyclo[4.4.0]dec-3-yl group, tricyclo[3.2.1.1^{3,7}]non-1-yl group, tricyclo[3.3.1.^{3,7}]dec-1-yl group, and tricyclo[3.3.1.1^{3,7}]dec-2-yl group.

The term "mono- or dicyclic aliphatic nitrogen-containing heterocyclic group" means a saturated aliphatic heterocyclic group which has at least one nitrogen atom as a ring atom and is mono- or dicyclic, and examples thereof include a group represented by: 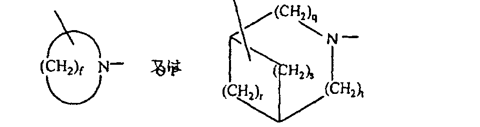 (wherein f represents an integer of 3 to 9; q, r and t are the same or different and represent an integer of 0 to 3; and s represents an integer of 1 to 4).

The term "lower alkoxycarbonyl group" as used herein means an alkoxycarbonyl group having the lower alkoxy group, namely an alkoxycarbonyl group having 2 to 7 carbon atoms, and examples thereof include methoxycarbonyl group, ethoxycarbonyl group, propoxycarbonyl group, isopropoxycarbonyl group, butoxycarbonyl group, isobutoxycarbonyl group, tert-butoxycarbonyl group, and pentyloxycarbonyl group.

The term "lower alkylcarbamoyl group" as used herein means a carbamoyl group which is mono-substituted with the lower alkyl group, and examples thereof include methylcarbamoyl group, ethylcarbamoyl group, propylcarbamoyl group, isopropylcarbamoyl group, butylcarbamoyl group, sec-butylcarbamoyl group, and tert-butylcarbamoyl group.

The term "di-lower alkylcarbamoyl group" as used herein means a carbamoyl group which is di-substituted with the lower alkyl group, and examples thereof include dimethylcarbamoyl group, diethylcarbamoyl group, ethylmethylcarbamoyl group, dipropylcarbamoyl group, methylpropylcarbamoyl group, and diisopropylcarbamoyl group.

The term "lower alkylsulfonylamino group" as used herein means a sulfonylamino group having the lower alkyl group, and examples thereof include methylsulfonylamino group, ethylsulfonylamino group, propylsulfonylamino group, isopropylsulfonylamino group, butylsulfonylamino group, sec-butylsulfonylamino group, and tert-butylsulfonylamino group.

The term "(lower alkylamino)sulfonylamino group" as used herein means a sulfonylamino group having the lower alkylamino group, and examples thereof include (methylamino)sulfonylamino group, (ethylamino)sulfonylamino group, (propylamino)sulfonylamino group, (isopropylamino)sulfonylamino group, (butylamino)sulfonylamino group, (sec-butylamino)sulfonylamino group, and (tert-butylamino)sulfonylamino group.

The term "(di-lower alkylamino)sulfonylamino group" as used herein means a sulfonylamino group having the di-lower alkylamino group, and examples thereof include (dimethylamino)sulfonylamino group, (diethylamino)sulfonylamino group, (ethylmethylamino)sulfonylamino group, (dipropylamino)sulfonylamino group, (methylpropylamino)sulfonylamino group, and (diisopropylamino)sulfonylamino group.

The term "(lower alkylcarbamoyl)amino group" as used herein means an amino group which is mono-substituted with the lower alkylcarbamoyl group, and examples thereof include (methylcarbamoyl)amino group, (ethylcarbamoyl)amino group, (propylcarbamoyl)amino group, (isopropylcarbamoyl)amino group, (butylcarbamoyl)amino group, (sec-butylcarbamoyl)amino group, and (tert-butylcarbamoyl)amino group.

The term "(di-lower alkylcarbamoyl)amino group" as used herein means an amino group which is mono-substituted with the di-lower alkylcarbamoyl group, and examples thereof include (dimethylcarbamoyl)amino group, (diethylcarbamoyl)amino group, (ethylmethylcarbamoyl)amino group, (dipropylcarbamoyl)amino group, (methylpropylcarbamoyl)amino group, and (diisopropylcarbamoyl)amino group.

The term "lower alkylcarbamoyloxy group" as used herein means an oxy group having the lower alkylcarbamoyl group, and examples thereof include methylcarbamoyloxy group, ethylcarbamoyloxy group, propylcarbamoyloxy group, isopropylcarbamoyloxy group, butylcarbamoyloxy group, sec-butylcarbamoyloxy group, and tert-butylcarbamoyloxy group.

The term "di-lower alkylcarbamoyloxy group" as used herein means an oxy group having the di-lower alkylcarbamoyl group, and examples thereof include dimethylcarbamoyloxy group, diethylcarbamoyloxy group, ethylmethylcarbamoyloxy group, dipropylcarbamoyloxy group, methylpropylcarbamoyloxy group, and diisopropylcarbamoyloxy group.

Examples of the "cycloalkyl group having 3 to 10 carbon atoms" include cyclopropyl group, cyclobutyl group, cyclopentyl group, cyclohexyl group, cycloheptyl group, cyclooctyl group, cyclononyl group, and cyclodecyl group.

The term "salt" of the compound represented by the general formula (III) means a conventional salt which is pharmaceutically acceptable, and examples thereof include salts, for example, base addition salt in a carboxyl group in case of having the carboxyl group, or an acid addition salt in an amino group in case of having the amino group or in a basic heterocycle in case of having the basic heterocycle.

Examples of the base addition salt include alkali metal salts such as sodium salt and potassium salt; alkali earth metal salts such as calcium salt and magnesium salt; ammonium salt; and organic amine salts such as trimethylamine salt, triethylamine salt, dicyclohexylamine salt, ethanolamine salt, diethanolamine salt, triethanolamine salt, procaine salt, and N,N'-dibenzylethylenediamine salt.

Examples of the acid addition salt include inorganic acid salts such as hydrochloride, sulfate, nitrate, phosphate, and perchlorate; organic acid salts such as maleate, fumarate, tartrate, citrate, ascorbate, and trifluoroacetate; and sulfonates such as methanesulfonate, isethionate, benzenesulfonate, and p-toluenesulfonate.

The term "ester" of the compound represented by the general formula (III) means a conventional ester which is pharmaceutically acceptable in a carboxyl group in case of having the carboxyl group, and examples thereof include ester with a lower alkyl group such as methyl group, ethyl group, propyl group, isopropyl group, butyl group, sec-butyl group, tert-butyl group, pentyl group, isopentyl group, neopentyl group, cyclopropyl group, cyclobutyl group, or cyclopentyl group, ester with an aralkyl group such as benzyl group or phenethyl group, ester with a lower alkenyl group such as allyl group or 2-butenyl group, ester with a lower alkoxyalkyl group such as methoxymethyl group, 2-methoxyethyl group, or 2-ethoxyethyl group, ester with a lower alkanoyloxyalkyl group such as acetoxymethyl group, pivaloyloxymethyl group, or 1-pivaloyloxyethyl group, ester with a lower alkoxycarbonylalkyl group such as methoxycarbonylmethyl group or isopropoxycarbonylmethyl group, ester with a lower carboxyalkyl group such as carboxymethyl group, ester with a lower alkoxycarbonyloxyalkyl group such as 1-(ethoxycarbonyloxy)ethyl group or 1-(cyclohexyloxycarbonyloxy)ethyl group, ester with a lower carbamoyloxyalkyl group such as carbamoyloxymethyl group, ester with phthalidyl, and ester with a (5-substituted-2-oxo-1,3-dioxol-4-yl)methyl group such as (5-methyl-2-oxo-1,3-dioxol-4-yl)methyl group.

Definitions of the respective substituents of the general formula (IV) described in claim 2 of the present specification are the same as in International Publication WO99/48492 (Japanese Unexamined Patent Publication No. 11-335355).

The term "halogen atom" as used herein means fluorine, chlorine, bromine or iodine atom. In R²³, R²⁴ and R²⁵, chlorine atom is preferable.

The term "lower alkyl group" as used herein means a straight or branched alkyl group having 1 to 6 carbon atoms, and examples thereof include methyl group, ethyl group, propyl group, isopropyl group, butyl group, isobutyl group, sec-butyl group, tert-butyl group, pentyl group, isopentyl group, tert-pentyl group, and hexyl group. Among them, a straight or branched alkyl group having 1 to 4 carbon atoms is preferable, and a methyl group or an ethyl group is more preferable.

The term "lower alkoxy group" as used herein means an alkyloxy group, an alkyl moiety of which is the above-defined "lower alkyl group". Specific examples thereof include methoxy group, ethoxy group, propoxy group, isopropyloxy group, and tert-butoxy group.

The term "lower alkylthio group" as used herein means an alkylthio group, an alkyl moiety of which is the above-defined "lower alkyl group". Specific examples thereof include methylthio group, ethylthio group, propylthio group, isopropylthio group, and tert-butylthio group. The alkyl moiety is preferably a straight or branched alkyl group having 1 to 4 carbon atoms, and more preferably a methylthio group.

The term "lower alkanoyl group" as used herein means an alkylcarbonyl group, an alkyl moiety of which is the above-defined "lower alkyl group". Specific examples thereof include acetyl group, propionyl group, butyryl group, isobutyryl group, and pivaloyl group. The alkyl moiety is preferably a straight or branched alkyl group having 1 to 4 carbon atoms, and an acetyl group is more preferable in R²⁵.

The term "lower alkylsulfonyl group" as used herein means an alkylsulfonyl group, an alkyl moiety of which is the above-defined "lower alkyl group". Specific examples thereof include mesyl group, ethylsulfonyl group, propylsulfonyl group, isopropylsulfonyl group, and tert-butylsulfonyl group. The alkyl moiety is preferably a straight or branched alkyl group having 1 to 4 carbon atoms, and a mesyl group is more preferable in R²⁵.

The term "lower alkanoyloxy group" as used herein means an alkylcarbonyloxy group, an alkyl moiety of which is the above-defined "lower alkyl group". Specific examples thereof include acetoxy group, propionyloxy group, butyryloxy group, isobutyryloxy group, and pivaloyloxy group. The alkyl moiety is preferably a straight or branched alkyl group having 1 to 4 carbon atoms, and an acetoxy group is more preferable in R²⁵.

The term "lower alkoxycarbonyl group" as used herein means an alkyloxycarbonyl group, an alkyl moiety of which is the above-defined "lower alkyl group". Specific examples thereof include methoxycarbonyl group, ethoxycarbonyl group, propoxycarbonyl group, isopropyloxycarbonyl group, and tert-butoxycarbonyl group. The alkyl moiety is preferably a straight or branched alkyl group having 1 to 4 carbon atoms. A methocycarbonyl group is more preferable in R²⁵, and a tert-butoxycarbonyl group is more preferable in R²⁷.

The term "lower alkyl group which may be substituted with a hydroxyl group" is a lower alkyl group in which the above-defined "lower alkyl group" may be substituted with one or plural hydroxyl groups, and includes a non-substituted alkyl group. Specific examples thereof include methyl group, ethyl group, propyl group, isopropyl group, hydroxymethyl group, 1,2-dihydroxyethyl group, and 2-(hydroxymethyl)butyl group. In R²¹ and R²², methyl, ethyl, propyl, isopropyl and hydroxymethyl groups are preferable, and methyl and ethyl groups are more preferable.

The term "lower alkyl group which may be substituted with a lower alkoxy group" is the above-defined "lower alkyl group" which may be substituted with the above-defined "lower alkoxy group", and includes a non-substituted alkyl group. Specific examples thereof include methyl group, ethyl group, methoxymethyl group, ethoxymethyl group, and 2-(methoxymethyl)butyl group. The alkyl moiety as a stock thereof is preferably a straight alkyl group having 1 to 4 carbon atoms, and a methoxymethyl group is more preferably in X²⁰.

The term "lower alkoxy group which may be substituted with a lower alkoxy group" is the above-defined "lower alkoxy group" which may be substituted with the above-defined "lower alkoxy group", and includes a non-substituted alkoxy group. Specific examples thereof include methoxy group, ethoxy group, methoxymethoxy group, methoxyethoxy group, and 2-(methoxymethyl)butyloxy group. The alkyl moiety as a stock thereof is preferably a straight or branched alkyl group having 1 to 4 carbon atoms, and methoxy group and methoxymethoxy group are more preferable in R²⁵.

The term "lower alkyl group which may be substituted with any one of lower alkoxy group which may be substituted with a lower alkoxy group, halogen atom, hydroxyl group and lower alkanoyloxy group" is a lower alkyl group which may be substituted with one or plural substituent, which is the the same or different, and the substituent is selected from the group consisting of the above-defined "lower alkoxy groups which may be substituted with a lower alkoxy group", the above-defined "halogen atom", a hydroxyl group and the above-defined "lower alkanoyloxy group", and the lower alkyl group includes a non-substituted alkyl group. Specific examples thereof include methyl group, ethyl group, propyl group, isopropyl group, tert-butyl group, hydroxymethyl group, 2-hydroxyethyl group, 1,2-dihydroxyethyl group, acetoxymethyl group, pivaloyloxymethyl group, bromomethyl group, trifluoromethyl group, methoxymethoxymethyl group, and methoxyethoxymethyl group. The alkyl moiety as a stock thereof is preferably a straight or branched alkyl group having 1 to 4 carbon atoms. In R²⁵, methyl group, ethyl group, propyl group, isopropyl group, tert-butyl group, hydroxymethyl group, acetoxymethyl group, trifluoromethyl group and methoxymethoxymethyl group are more preferable, and ethyl group is still more preferable.

The term "lower alkylamino group" as used herein means a monoalkylamino group, an alkyl moiety of which is the above-defined "lower alkyl group". Specific examples thereof include methylamino group, ethylamino group, propylamino group, isopropylamino group, and tert-butylamino group. The alkyl moiety is preferably a straight or branched alkyl group having 1 to 4 carbon atoms, and a methylamino group is more preferable in R²¹ and R²².

The term "di-lower alkylamino group" as used herein means a dialkylamino group, an alkyl moiety of which is the same or different and is the above-defined "lower alkyl group". Specific examples thereof include dimethylamino group, diethylamino group, methylethylamino group, and N-isopropyl-N-isobutylamino group. The alkyl moiety is preferably a straight or branched alkyl group having 1 to 4 carbon atoms, and a dimethylamino group is more preferable in R²¹, R²² and R²⁵.

The term "lower alkenyl group" as used herein means a straight alkenyl group having 1 to 6 carbon atoms, and examples thereof include vinyl group, 1-propenyl group, 2-propenyl group, 1-butenyl group, 2-butenyl group, 3-butenyl group, 1,3-butadienyl group, 2,4-butadienyl group, 1-pentenyl, 1,3-pentadienyl group, and 1,3,5-hexatrienyl group. In X²⁰, a vinyl group is preferable.

The term "aryl group" as used herein means an aromatic hydrocarbon group having 6 to 18 carbon atoms, and examples thereof include phenyl group, naphthyl group, anthryl group, indenyl group, azulenyl group, fluorenyl group, phenanthryl group, and pyrenyl group. In the ring A, phenyl and naphthyl groups are preferable and a phenyl group is more preferable. In the ring G and R²⁶, a phenyl group is preferable. When the ring G is a phenyl group, preferable substitution position of the substituent R²⁵ is para-position.

The term "heterocyclic group" as used herein is a cyclic compound group which has one or plural kinds of hetero atoms or one or plural hetero atoms selected from among oxygen atom, nitrogen atom and sulfur atom, the number of atoms constituting the ring being 5 to 20. Specific examples thereof include pyridyl group, pyrazinyl group, pyrimidinyl group, pyrrolyl group, thienyl group, furyl group, imidazolyl group, pyrazolyl group, oxazolyl group, thiazolyl group, quinolyl group, isoquinolyl group, indolyl group, benzofuranyl group, benzimidazolyl group, imidazolidinyl group, indolinyl group, pyrrolidinyl group, pyrrolinyl group, piperidinyl group, piperazinyl group, chromanyl group, morpholinyl group, phthalazinyl group, naphthylidinyl group, quinazolinyl group, quinoxalyl group, cinnolinyl group, pteridinyl group, 4H-quinolizinyl group, carbazolyl group, 1,3,5-triazinyl group, 2,3-dihydrobenzofuranyl group, 5,6,7,8-tetrahydroquinolyl group, 5,6,7,8-tetrahydroacrylidinyl group, and 2,3-dihydro-1H-cyclopenta[b]quinolyl group. In the ring G, pyridyl, benzofuranyl and 2,3-dihydrobenzofuranyl groups are preferable, and a 2,3-dihydrobenzofuranyl group is more preferable. In the ring A, preferred is a cyclic compound group which has one or plural nitrogen atoms as a hetero atom, the number of atoms constituting the ring being 9 to 14, quinolyl, isoquinolyl, quinoxalyl, benzimidazolyl, 5,6,7,8-tetrahydroquinolyl, 5,6,7,8-tetrahydroacrylidinyl and 2,3-dihydro-1H-cyclopenta[b]quinolyl groups are more preferable, quinolyl, 5,6,7,8-tetrahydroacrylidinyl and 2,3-dihydro-1H-cyclopenta[b]quinolyl groups are still more preferable, and a quinolyl group is most preferable. When the ring A is a quinolyl group, R²¹ is preferably an amino group and is substituted at the 4-position and R²² is preferably a lower alkyl group and is substituted at the 2-position, and -NHCO- is preferably substituted at the 6-position.

The term "cycloalkyl group" as used herein means a saturated cycloalkyl group having 3 to 8 carbon atoms, for example, cyclopropyl group, cyclobutyl group, cyclopentyl group, cyclohexyl group, cycloheptyl group, and cyclooctyl group. In the ring G, a cyclohexyl group is preferable.

The term "fused aryl group" as used herein is a cyclic compound group in which the above-defined "aryl group" is fused with the above-defined "cycloalkyl group", the number of atoms constituting the ring being 5 to 18. Specific examples thereof include indanyl group, 5,6,7,8-tetrahydro-2-naphthyl group, 5,6,7,8-tetrahydro-3-naphthyl group, 1,2,3,4-tetrahydro-2-naphthyl group, 5,6,7,8-tetrahydro-2-anthryl group, and 1,2,3-trihydroazulenyl group. In the ring G, a 5,6,7,8-tetrahydro-2-naphtyl group is preferable.

The term "protected amino group" as used herein is an amino group protected with an amino protective group used in common chemical synthesis and specific examples of the protective group include formyl group, acetyl group, benzoyl group, benzyloxycarbonyl group, methoxycarbonyl group, tert-butoxycarbonyl group, phthaloyl group, benzyl group, and tosyl group.

The term "protective group of carboxy group" as used herein is a protective group of a carboxy group, which is used in common chemical synthesis, and specific examples thereof include methyl group, methoxyethoxymethyl group, phenacyl group, phthalimidemethyl group, ethyl group, 2,2,2-trichloroethyl group, 2-methylthioethyl group, tert-butyl group, benzyl group, p-nitrobenzyl group, p-methoxybenzyl group, and tert-butyldimethylsilyl group. The term "protective group of hydroxyl group" means a protective group of a hydroxyl group, which is used in common chemical synthesis, and specific examples thereof include trimethylsilyl group, tert-butyldimethylsilyl group, methyl group, benzyl group, p-methoxybenzyl group, tert-butyl group, trityl group, tetrahydropyranyl group, methoxymethyl group, methoxyethoxyethyl group, acetyl group, and benzoyl group.

Preferable aspects in definitions of the respective symbols of the above-mentioned general formula (IV) will be described below. In the ring G, an aryl group is preferable. In R²⁵, preferred are a halogen atom; a lower alkyl group which may be substituted with any one of a lower alkoxy group which may be substituted with a lower alkoxy group, a halogen atom, a hydroxyl group and a lower alkanoyloxy group; a lower alkoxy group which may be substituted with a lower alkoxy group; a nitro group; a cyano group; and a lower alkanoyl group, more preferably a lower alkyl group which may be substituted with any one of a lower alkoxy group which may be substituted with a lower alkoxy group, a halogen atom, a hydroxyl group or a lower alkanoyloxy group. In t, an integer of 0 or 1 to 2 is preferable and 1 is more preferable. In W, a single bond and -O- are preferable and -O- is more preferable. When W is -O-, an integer of 1 to 7 is preferable and 1 is more preferable in m, and 0 is preferable in g. When W is a single bond, m + g is preferably 2.

In the compound represented by the above-mentioned general formula (IV), various isomers exist. In case E form and Z form exist as a geometrical isomer and an asymmetric carbon atom exists, an enantiomer and a diastereomer as a stereoisomer based on them exist. In some cases, a tautomer can exist. Therefore, these isomers and a mixture thereof are also included in the present invention.

The term "pharmacologically acceptable salt thereof" as used herein may be any salt as far as it can form a non-toxic salt with the compound represented by the above-mentioned general formula (IV), and can be obtained by reacting with inorganic acids such as hydrochloric acid, sulfuric acid, phosphoric acid, and hydrobromic acid; organic acids such as oxalic acid, malonic acid, citric acid, fumaric acid, lactic acid, malic acid, succinic acid, tartaric acid, acetic acid, gluconic acid, ascorbic acid, methylsulfonic acid, and benzylsulfonic acid; inorganic bases such as sodium hydroxide, potassium hydroxide, calcium hydroxide, magnesium hydroxide, and ammonium hydroxide; organic bases such as methylamine, diethylamine, triethylamine, triethanolamine, ethylenediamine, tris(hydroxymethyl)methylamine, guanidine, choline, and cinchonine; and amino acids such as lysine, arginine, and alanine. In the present invention, a hydrated compound or a hydrate and a solvate of the respective compounds are also included.

In the present invention, a prodrug and a metabolite of the respective compounds are also included. The term "prodrug" as used herein means a derivative of the compound of the present invention, which has a chemically or metabolically decomposable group and exhibits original medicament efficacy after it was administered to the living body and turned into an original compound, and also contains a complex and a salt free from a covalent bond.

In the compounds of the general formulas (I), (II), (III) and (IV) described in claim 2, a compound may exist as a cis (Z form) isomer or a trans (E form) isomer, and each isomer and a mixture thereof are also included in the present invention.

Specific examples of the compound of the general formula (I) used as a nociceptin antagonist include the following compounds:
cis-N-amidino-2-[2-(4-chlorostyryl)-6-methoxyquinazolin-4-yl]aminocyclohexylamine dihydrochloride,
cis-N-amidino-2-[2-(4-chlorobenzoylamino)-6-methoxyquinazolin-4-yI]aminocyclohexylamine dihydrochloride,
cis-N-acetoimide-2-[2-(4-chlorostyryl)-6-methoxyquinazolin-4-yl]aminocyclohexylamine dihydrochloride,
cis-4-guanidinomethyl-N-{2-[2-(2-pyridyl)ethenyl]-6-methoxyquinazolin-4-yl}cyclohexylamine trihydrochloride,
N-2-(2-imidazolynyl)-N'-[2-(4-chlorostyryl)-6-methylquinazolin-4-yl]-1,4-cyclohexanediamine
   dihydrochloride,
N-amidino-N'-[2-(4-chlorostyryl)quinazolin-4-yl]-1,4-butanediamine dihydrochloride,
N-amidino-N'-[2-(4-chlorostyryl)quinazolin-4-yl]-1,5-pentanediamine dihydrochloride,
N-amidino-N'-[2-(4-chlorostyryl)-6-methylquinazolin-4-yl]-1,6-hexanediamine dihydrochloride,
N-amidino-N'-[2-(4-chlorostyryl)quinazolin-4-yl]-1,3-propanediamine dihydrochloride,
N-amidino-N'-[2-(4-chlorostyryl)-6-methylquinazolin-4-yl]-1,4-butanediamine dihydrochloride,
N-amidino-N'-[2-(4-chlorostyryl)-6-methylquinazolin-4-yl]-1,5-pentanediamine dihydrochloride,
cis-N-amidino-2-[2-(4-chlorostyryl)-6-methylquinazolin-4-yl]aminocyclohexylamine dihydrochloride,
(1R,2S)-N'-amidino-2-[2-(4-chlorostyryl)-6-methylquinazolin-4-yl]aminocyclohexylamine dihydrochloride,
(1S,2R)-N-amidino-2-[2-(4-chlorostyryl)-6-methylquinazolin-4-yl]aminocyclohexylamine dihydrochloride,
N-amidino-N'-[6-t-butyl-2-(4-chlorostyryl)quinazolin-4-yl]-1,6-hexanediamine dihydrochloride,
N-amidino-N'-[2-(4-chlorostyryl)-6-methoxyquinazolin-4-yl]-1,6-hexanediamine dihydrochloride,
N-amidino-N'-[2-(4-chlorostyryl)-6,7-dimethylquinazolin-4-yl)-1,5-pentanediamine dihydrochloride,
N-amidino-N'-[2-(4-chlorostyryl)-6-isopropylquinazolin-4-yl]-1,6-hexanediamine dihydrochloride,
cis-N-amidino-N'-[2-(4-chlorostyryl)-6-methylquinazolin-4-yl]-1,4-cyclohexanediamine dihydrochloride,
cis-N-amidino-2-[2-(4-chlorostyryl)-6,7-dimethylquinazolin-4-yl]aminocyclohexylamine dihydrochloride,
cis-N-amidino-3-[2-(4-chlorostyryl)-6,7-dimethylquinazolin-4-yl]aminocyclohexylamine dihydrochloride,
trans-N-amidino-3-[2-(4-chlorostyryl)-6-methylquinazolin-4-yl]aminocyclohexylamine dihydrochloride,
N-amidino-N'-{6-methoxy-2-[2-(2-pyridyl)ethenyl]quinazolin-4-yl}-1,6-hexanediamine dihydrochloride,
(1R,2S)-N-amidino-2-[2-(4-chlorostyryl)-6-methoxyquinazolin-4-yl]aminocyclohexylamine dihydrochloride,
(1S,2R)-N-amidino-2-[2-(4-chlorostyryl)-6-methoxyquinazolin-4-yl)aminocyclohexylamine dihydrochloride,
N-amidino-N'-[2-(4-chlorostyryl)-quinazolin-4-yl]-1,4-bis(aminomethyl)cyclohexane dihydrochloride,
N-amidino-N'-[2-(4-chlorostyryl)benz[g]quinazolin-4-yl]-1,6-hexanediamine dihydrochloride,
cis-N-amidino-2-[2-(4-chlorostyryl)-6-isopropylquinazolin-4-yl]aminocyclohexylamine dihydrochloride,
N-amidino-N'-[2-(4-chlorostyryl)-6-methylquinazol in-4-yl]-1,4-bis(aminomethyl)cyclohexane dihydrochloride,
cis-N-amidino-2-[2-(4-chlorostyryl)-6-hydroxyquinazolin-4-yl]aminocyclohexylamine dihydrochloride,
cis-N-amidino-2-{6-methyl-2-[2-(4-pyridyl)ethenyl]quinazolin-4-yl}aminocyclohexylamine trihydrochloride,
cis-N-amidino-2-[2-(4-chlorobenzoylamino)-6-methylquinazolin-4-yl]aminocyclohexylamine dihydrochloride,
cis-N-amidino-2-[2-(4-chlorostyryl)-6-ethoxyquinazolin-4-yl]aminocyclohexylamine dihydrochloride,
cis-N-amidino-2-{6-methoxy-2-[2-(3-pyridyl)ethenyl]quinazolin-4-yl}aminocyclohexylamine trihydrochloride,
(1R,2S)-cis-N-amidino-2-{6-methoxy-2-[2-(3-pyridyl)ethenyl]quinazolin-4-yl}aminocyclohexylamine trihydrochloride,
(1S,2R)-cis-N-amidino-2-{6-methoxy-2-[2-(3-pyridyl)ethenyl]quinazoiin-4-yl}aminocyclohexylamine trihydrochloride,
(1R,2S)-N-amidino-2-[2-(4-chlorostyryl)-6-methoxyquinazolin-4-yl]aminocyclopentylamine dihydrochloride,
(1S,2R)-N-amidino-2-[2-(4-chlorostyryl)-6-methoxyquinazolin-4-yl]aminocyclopentylamine dihydrochloride,
cis-N-amidino-2-{6-methoxy-2-[2-(2-pyridyl)ethenyl]quinazolin-4-yl}aminocyclohexylamine trihydrochloride,
(1R,2S)-cis-N-amidino-2-{6-methoxy-2-[2-(2-pyridyl)ethenyl]quinazolin-4-yl}aminocyclohexylamine trihydrochloride,
(1S,2R)-cis-N-amidino-2-{6-methoxy-2-[2-(2-pyridyl)ethenyl]quinazolin-4-yl}aminocyclohexylamine trihydrochloride,
cis-N-amidino-2-{6-methoxy-2-[2-(4-pyridyl)ethenyl]quinazolin-4-yl}aminocyclohexylamine trihydrochloride,
cis-N-amidino-2-[6-methoxy-2-(2-methoxystyryl)quinazolin-4-yl]aminocyclohexylamine dihydrochloride,
cis-N-amidino-N'-{6-methoxy-2-[2-(2-pyridyl)ethenyl]quinazolin-4-yl}-1,4-bis(aminomethyl)cyclohexane trihydrochloride,
trans-N-amidino-N'-{6-methoxy-2-[2-(2-pyridyl)ethenyl]quinazolin-4-yl}-1,4-bis(aminomethyl)cyclohexane trihydrochloride,
N-amidino-N'-{6-methyl-2-[2-(2-pyridyl)ethenyl]quinazolin-4-yl}-1,6-hexanediamine trihydrochloride,
N-amidino-N'-{6-methyl-2-[2-(2-pyridyl)ethenyl]quinazolin-4-yl}-1,8-octanediamine trihydrochloride,
N-amidino-6-{6-methoxy-2-[2-(2-pyridyl)ethenyl]quinazolin-4-yl}amino-heptylamine trihydrochloride,
N-[2-(4-chlorostyryl)quinazolin-4-yl]-N'-(2-pyrimidyl)piperazine dihydrochloride,
cis-N-[2-(4-chlorostyryl)-6-methoxyquinazolin-4-yl]-2-guanidinomethylcyclohexylamine dihydrochloride,
N-2-(2-imidazolynyl)-N'-[2-(4-chlorostyryl)-6-methylquinazolin-4-yl]-1,6-hexanediamine dihydrochloride,
N-[2-(4-chlorostyryl)quinazolin-4-yl]-1,2-ethanediamine dihydrochloride,
N-amidino-N'-[2-(4-chlorostyryl)quinazolin-4-yl]-1,6-hexanediamine dihydrochloride,
N-amidino-N'-(2-(4-chlorostyryl)-6-methylquinolin-4-yl]-1,6-hexanediamine dihydrochloride,
N-amidino-N'-[2-(4-chlorostyryl)quinolin-4-yl]-1,6-hexanediamine dihydrochloride,
cis-N-amidino-2-[2-(4-chlorostyryl)-6-methylquinolin-4-yl]aminocyclohexylamine dihydrochloride,
cis-N-amidino-2-[2-(4-chlorostyryl)-6-methoxyquinolin-4-yl]aminocyclohexylamine dihydrochloride,
cis-N-amidino-2-[3-(4-chlorostyryl)isoquinolin-1-yl]aminocyclohexylamine dihydrochloride,
N-amidino-4-{6-methyl-2-[2-(2-pyridyl)ethenyl]quinazolin-4-yl}aminomethyl-benzylamine trihydrochloride,
N-(N-isobutyl-N'-phenyl)amidino-N'-{6-methoxy-2-[2-(2-pyridyl)ethenyl]quinazolin-4-yl}-1,6-hexanediamine trihydrochloride,
2-guanidinoethoxy-N-{6-methyl-2-[2-(2-pyridyl)ethenyl]quinazolin-4-yl}ethylamine trihydrochloride,
N-(N-methyl-N'-phenyl)amidino-N'-{6-methoxy-2-[2-(2-pyridyl)ethenyl]quinazolin-4-yl}-1,6-hexanediamine trihydrochloride,
N-(N-ethyl-N'-methyl)amidino-N'-{6-methoxy-2-(2-(2-pyridyl)ethenyl]quinazolin-4-yl}-1,6-hexanediamine trihydrochloride,
2-guanidinopropyloxy-N-{6-methyl-2-[2-(2-pyridyl)ethenyl]quinazolin-4-yl}ethylamine trihydrochloride,
3-guanidinoethoxy-N-{6-methyl-2-[2-(2-pyridyl)ethenyl]quinazolin-4-yl}propylamine trihydrochloride,
N-amidino-2-{6-methoxy-2-[2-(2-pyridyl)ethenyl]quinazolin-4-yl}aminoethyl-phenylethylamine trihydrochloride,
trans-4-guanidinomethyl-cis-2-methyl-N-{6-methoxy-2-[2-(2-pyridyl)ethenyl]quinazolin-4-yl}cyclohexylamine trihydrochloride,
cis-4-guanidinomethyl-cis-2-methyl-N-{6-methoxy-2-[2-(2-pyridyl)ethenyl]quinazolin-4-yl}cyclohexylamine trihydrochloride,
(1R,2S)-N-amidino-2-[2-(4-chlorobenzoylamino)-6-methoxyquinazolin-4-yl]aminocyclohexylamine dihydrochloride, and
(1R,2S)-N-amidino-2-{[2-(4-chlorobenzoylamino)-6-methoxyquinazolin-4-yl]amino}cyclohexylamine dihydrochloride.

Specific examples of the compound of the general formula (II) used as the nociceptin antagonist include compounds in the Examples described hereinafter.

Specific examples of the compound of the general formula (III) used as the nociceptin antagonist include the following compounds:
N-(4-amino-2-methyl-6-quinolyl)-2-[(4-ethylphenoxy)methyl]benzamide hydrochloride,
N-(4-amino-2-methyl-6-quinolyl)-2-[(2,4-dichlorophenoxy)methyl]benzamide hydrochloride,
N-(4-amino-2-methyl-6-quinolyl)-2-(phenoxymethyl)benzamide hydrochloride,
N-(4-amino-2-methyl-6-quinolyl)-2-[(4-methoxyphenoxy)methyl]benzamide hydrochloride,
N-(4-amino-2-methyl-6-quinolyl)-2-[(3,5-dimethylphenoxy)methyl]benzamide hydrochloride,
N-(4-amino-2-methyl-6-quinolyl)-2-[(3,4-dimethoxyphenoxy)methyl]benzamide hydrochloride,
N-(4-amino-2-methyl-6-quinolyl)-2-[(4-nitrophenoxy)methyl]benzamide,
N-(4-amino-2-methyl-6-quinolyl)-2-[(2,3-dimethoxyphenoxy)methyl]benzamide hydrochloride,
N-(4-amino-2-methyl-6-quinolyl)-2-[(3-methylphenoxy)methyl]benzamide,
N-(4-amino-2-methyl-6-quinolyl)-2-[(3,5-dimethoxyphenoxy)methyl]benzamide hydrochloride,
N-(4-amino-2-methyl-6-quinolyl)-2-[(4-chlorophenoxy)methyl]benzamide hydrochloride,
N-(4-amino-2-methyl-6-quinolyl)-2-[(4-acetylphenoxy)methyl]benzamide hydrochloride,
N-(4-amino-2-methyl-6-quinolyl)-2-[(4-hydroxyphenoxy)methyl]benzamide hydrochloride,
N-(4-amino-2-methyl-6-quinolyl)-2-[(4-methoxymethoxyphenoxy)methyl]benzamide hydrochloride,
N-(4-amino-2-methyl-6-quinolyl)-2-[(3-methoxyphenoxy)methyl]benzamide hydrochloride,
N-(4-amino-2-methyl-6-quinolyl)-2-[(4-cyanophenoxy)methyl]benzamide hydrochloride,
N-(4-amino-2-methyl-6-quinolyl)-2-[(4-methylphenoxy)methyl]benzamide hydrochloride,
N-(4-amino-2-methyl-6-quinolyl)-2-[(4-trifluoromethylphenoxy)methyl]benzamide hydrochloride,
N-(4-amino-2-methyl-6-quinolyl)-2-[(3-nitrophenoxy)methyl]benzamide hydrochloride,
N-(4-amino-2-methyl-6-quinolyl)-2-[(2-nitrophenoxy)methyl]benzamide hydrochloride,
N-(4-amino-2-methyl-6-quinolyl)-2-[(4-acetoxyphenoxy)methyl]benzamide hydrochloride,
N-(4-amino-2-methyl-6-quinolyl)-2-[(2-methoxyphenoxy)methyl]benzamide hydrochloride,
N-(4-amino-2-methyl-6-quinolyl)-2-[(4-aminophenoxy)methyl]benzamide dihydrochloride,
N-(4-amino-2-methyl-6-quinolyl)-2-[(3-chlorophenoxy)methyl]benzamide hydrochloride,
N-(4-amino-2-methyl-6-quinolyl)-2-[(4-fluorophenoxy)methyl]benzamide hydrochloride,
N-(4-amino-2-methyl-6-quinolyl)-2-[(3,4-dichlorophenoxy)methyl]benzamide hydrochloride,
N-(4-amino-2-methyl-6-quinolyl)-2-[(2-chlorophenoxy)methyl]benzamide hydrochloride,
N-(4-amino-2-methyl-6-quinolyl)-2-[(4-dimethylaminophenoxy)methyl]benzamide dihydrochloride,
N-(4-amino-2-methyl-6-quinolyl)-2-[(4-tert-butylphenoxy)methyl]benzamide hydrochloride,
N-(4-amino-2-methyl-6-quinolyl)-2-(4-biphenylyloxymethyl)benzamide hydrochloride,
N-(4-amino-2-methyl-6-quinolyl)-2-[(4-isopropylphenoxy)methyl]benzamide hydrochloride,
N-(4-amino-2-methyl-6-quinolyl)-2-[(4-nitrophenoxy)methyl]benzamide hydrochloride,
N-(4-amino-2-methyl-6-quinolyl)-2-[(4-bromophenoxy)methyl]benzamide hydrochloride,
N-(4-amino-2-methyl-6-quinolyl)-2-[(4-propylphenoxy)methyl]benzamide hydrochloride,
N-(4-amino-2-methyl-6-quinolyl)-2-((3-fluorophenoxy)methyl]benzamide hydrochloride,
N-(4-amino-2-methyl-6-quinolyl)-2-[(3-trifluoromethylphenoxy)methyl]benzamide hydrochloride,
Methyl 4-{2-[N-(4-amino-2-methyl-6-quinolyl)carbamoyl ]benzyloxy} benzoate hydrochloride,
N-(4-amino-2-methyl-6-quinolyl)-2-[(4-iodophenoxy)methyl]benzamide,
N-(4-amino-2-methyl-6-quinolyl)-2-(3-pyridyloxymethyl)benzamide hydrochloride,
4-{2-[(4-amino-2-methyl-6-quinolyl)carbamoyl]benzyloxy}benzoic acid hydrochloride,
N-(4-amino-2-methyl-6-quinolyl)-2-[(3-cyanophenoxy)methyl]benzamide hydrochloride,
N-(4-amino-2-methyl-6-quinolyl)-2-[(4-mesylphenoxy)methyl]benzamide hydrochloride,
N-(4-amino-2-methyl-6-quinolyl)-2-[(2-chloro-4-ethylphenoxy)methyl]benzamide hydrochloride,
N-(4-amino-2-methyl-6-quinolyl)-2-[(4-chloro-3-methylphenoxy)methyl]benzamide hydrochloride,
N-(4-amino-2-methyl-6-quinolyl)-2-[(2-chloro-4-methylphenoxy)methyl]benzamide hydrochloride,
N-(4-amino-2-methyl-6-quinolyl)-2-[(4-ethylphenoxy)methyl]benzamide,
N-(4-amino-2-methyl-6-quinolyl)-2-[(4-chloro-3-methylphenoxy)methyl]benzamide,
4-{2-[(4-amino-2-methyl-6-quinolyl)carbamoyl]benzyloxy}benzyl acetic acid hydrochloride,
N-(4-amino-2-methyl-6-quinolyl)-2-[(4-hydroxymethylphenoxy)methyl]benzamide hydrochloride, and
N-(4-amino-2-methyl-6-quinolyl)-2-[(4-ethylphenoxy)methyl]benzamide hydrochloride monohydrate.

Specific examples of the compound of the general formula (IV) used as the nociceptin antagonist include the following compounds:
1-(1-cyclohexylmethyl-4-piperidyl)-5-methyl-1,3-dihydro-2H-benzimidazol-2-one,
1-(1-cyclopropylmethyl-4-piperidyl)-5-methyl-1,3-dihydro-2H-benzimidazol-2-one,
1-(1-cyclooctylmethyl-4-piperidyl)-5-methyl-1,3-dihydro-2H-benzimidazol-2-one,
1-[1-(bicyclo(4.4.0]dec-3-ylmethyl)-4-piperidyl]-5-methyl-1,3-dihydro-2H-benzimidazol-2-one,
1-[1-(bicyclo[4.4.0]dec-2-ylmethyl)-4-piperidyl]-5-methyl-1,3-dihydro-2H-benzimidazol-2-one,
1-(1-cyclooctylmethyl-4-piperidyl)-1,3-dihydro-2H-benzimidazol-2-one,
1-[1-(1-cyclohexylethyl)-4-piperidyl]-5-methyl-1,3-dihydro-2H-benzimidazol-2-one,
1-[1-(1-cyclohexylethyl)-4-piperidyl]-1,3-dihydro-2H-benzimidazol-2-one,
1-[1-(tricyclo[3.3.1.1^{3,7}]dec-2-ylmethyl)-4-piperidyl]-1,3-dihydro-2H-benzimidazol-2-one,
1-[1-(bicyclo[4.4.0]dec-3-ylmethyl)-4-piperidyl]-1,3-dihydro-2H-benzimidazol-2-one,
1-(1-cyclononylmethyl-4-piperidyl)-1,3-dihydro-2H-benzimidazol-2-one,
1-(1-cyclooctylmethyl-4-piperidyl)-3-(3-hydroxypropyl)-1,3-dihydro-2H-benzimidazol-2-one,
1-(1-cyclooctylmethyl-4-piperidyl)-3-propargyl-1,3-dihydro-2H-benzimidazol-2-one,
1-(1-cyclodecylmethyl-4-piperidyl)-1,3-dihydro-2H-benzimidazol-2-one,
1-(1-cyclooctylmethyl-4-piperidyl)-3-(4-pyridylmethyl)-1,3-dihydro-2H-benzimidazol-2-one hydrochloride,
1-(1-cyclooctylmethyl-4-piperidyl)-3-methyl-1,3-dihydro-2H-benzimidazol-2-one,
1-(1-cyclooctylmethyl-4-piperidyl)-3-(3-pyridylmethyl)-1,3-dihydro-2H-benzimidazol-2-one hydrochloride,
1-(1-cyclooctylmethyl-4-piperidyl)-3-(2-pyridylmethyl)-1,3-dihydro-2H-benzimidazol-2-one hydrochloride,
1-(1-cycloheptyl-4-piperidyl)-1,3-dihydro-2H-benzimidazol-2-one,
1-[1-(bicyclo[3.2.1]oct-3-ylmethyl)-4-piperidyl]-1,3-dihydro-2H-benzimidazol-2-one,
1-[1-(1-cyclooctenylmethyl)-4-piperidyl]-1,3-dihydro-2H-benzimidazol-2-one,
1-[1-(1-cyclodecenylmethyl)-4-piperidyl]-1,3-dihydro-2H-benzimidazol-2-one,
1-[1-(1-ethylcyclooctylmethyl)-4-piperidyl]-1,3-dihydro-2H-benzimidazol-2-one,
1-(1-cyclooctylmethyl-4-piperidyl)-3-ethyl-1,3-dihydro-2H-benzimidazol-2-one,
1-(1-cyclooctylmethyl-4-piperidyl)-3-isopropyl-1,3-dihydro-2H-benzimidazol-2-one,
1-(1-cyclooctylmethyl-4-piperidyl)-3-isobutyl-1,3-dihydro-2H-benzimidazol-2-one,
1-[1-(2-methylenecyclooctylmethyl)-4-piperidyl]-1,3-dihydro-2H-benzimidazol-2-one,
1-[1-(2-methylcyclooctylmethyl)-4-piperidyl]-1,3-dihydro-2H-benzimidazol-2-one,
1-(1-cyclooctylmethyl-4-piperidyl)-3-propyl-1,3-dihydro-2H-benzimidazol-2-one,
1-(1-cyclooctylmethyl-4-piperidyl)-3-(2-hydroxyethyl)-1,3-dihydro-2H-benzimidazol-2-one,
1-(1-cyclooctylmethyl-4-piperidyl)-3-methoxymethyl-1,3-dihydro-2H-benzimidazol-2-one,
1-(1-cyclooctylmethyl-4-piperidyl)-3-(2-methoxyethyl)-1,3-dihydro-2H-benzimidazol-2-one,
1-(1-cyclooctylmethyl-4-piperidyl)-3-(2-dimethylaminoethyl)-1,3-dihydro-2H-benzimidazol-2-one dihydrochloride,
1-(1-cyclooctylmethyl-4-piperidyl)-3-(2-diethylaminoethyl)-1,3-dihydro-2H-benzimidazol-2-one dihydrochloride,
1-[(3RS,4RS)-1-cyclooctylmethyl-3-methyl-4-piperidyl]-1,3-dihydro-2H-benzimidazol-2-one,
1-[(3RS,4RS)-1-cyclooctylmethyl-3-methyl-4-piperidyl]-1,3-dihydro-2H-benzimidazol-2-one,
1-[(3RS,4SR)-1-cyclooctylmethyl-3-methyl-4-piperidyl]-1,3-dihydro-2H-benzimidazol-2-one,
1-[(3RS,4RS)-1-cyclooctylmethyl-3-ethoxycarbonyl-4-piperidyl]-1,3-dihydro-2H-benzimidazol-2-one,
1-[(3RS,4SR)-1-cyclooctylmethyl-3-ethoxycarbonyl-4-piperidyl]-1,3-dihydro-2H-benzimidazol-2-one,
1-[(3RS,4RS)-1-cyclooctylmethyl-3-hydroxymethyl-4-piperidyl]-1,3-dihydro-2H-benzimidazol-2-one,
1-[(3RS,4SR)-1-cyclooctylmethyl-3-hydroxymethyl-4-piperidyl]-1,3-dihydro-2H-benzimidazol-2-one,
1-[(3RS,4RS)-1-cyclooctylmethyl-3-ethoxycarbonyl-4-piperidyl]-3-ethyl-1,3-dihydro-2H-benzimidazol-2-one,
1-[(3RS,4RS)-1-cyclooctylmethyl-3-hydroxymethyl-4-piperidyl]-3-ethyl-1,3-dihydro-2H-benzimidazol-2-one,
1-[(3RS,4RS)-1-cyclooctylmethyl-3-hydroxymethyl-4-piperidyl]-3-propyl-1,3-dihydro-2H-benzimidazol-2-one,
1-[(3RS,4RS)-1-(bicyclo[4.4.0]dec-2-ylmethyl)-3-hydroxymethyl-4-piperidyl]-1,3-dihydro-2H-benzimidazol-2-one,
1-[(3RS,4RS)-1-cyclononylmethyl-3-hydroxymethyl-4-piperidyl]-1,3-dihydro-2H-benzimidazol-2-one,
1-[1-cyclooctylmethyl-3,3-bis(hydroxymethyl)-4-piperidyl]-1,3-dihydro-2H-benzimidazol-2-one,
1-[1-cyclooctylmethyl-3,3-bis(hydroxymethyl)-4-piperidyl]-ethyl-1,3-dihydro-2H-benzimidazol-2-one,
1-[(2RS,4RS)-1-cyclooctylmethyl-2-hydroxymethyl-4-piperidyl]-3-ethyl-1,3-dihydro-2H-benzimidazol-2-one,
1-[(3RS,4RS)-1-cyclooctylmethyl-3-methoxymethyl-4-piperidyl]-3-ethyl-1,3-dihydro-2H-benzimidazol-2-one,
1-[(3RS,4RS)-3-benzyloxymethyl-1-cyclooctylmethyl-4-piperidyl]-3-ethyl-1,3-dihydro-2H-benzimidazol-2-one,
1-[(3RS,4RS)-1-cyclooctylmethyl-3-hydroxymethyl-4-piperidyl]-3-(2-dimethylaminoethyl)-1,3-dihydro-2H-benzimidazol-2-one,
1-[(3RS,4RS)-1-cyclooctylmethyl-3-hydroxymethyl-4-piperidyl]-3-isopropyl-1,3-dihydro-2H-benzimidazol-2-one,
1-[(3RS,4RS)-1-cyclooctylmethyl-3-hydroxymethyl-4-piperidyl]-3-(methoxyethyl)-1,3-dihydro-2H-benzimidazol-2-one,
1-[1-(1-methylcyclooctylmethyl)-4-piperidyl)-3-propargyl-1,3-dihydro-2H-benzimidazol-2-one,
1-[1-(1-ethylcyclooctylmethyl)-4-piperidyl]-3-propargyl-1,3-dihydro-2H-benzimidazol-2-one,
1-[1-(1-propylcyclooctylmethyl)-4-piperidyl]-3-propargyl-1,3-dihydro-2H-benzimidazol-2-one,
1-[(3RS,4RS)-1-cyclooctylmethyl-3-dimethylaminomethyl-4-piperidyl)-3-ethyl-1,3-dihydro-2H-benzimidazol-2-one,
1-[(3RS,4RS)-1-cyclooctylmethyl-3-methylaminomethyl-4-piperidyl]-3-ethyl-1,3-dihydro-2H-benzimidazol-2-one,
1-[(3S^{*},4S^{*})-3-aminomethyl-1-cyclooctylmethyl-4-piperidyl]-3-ethyl-1,3-dihydro-2H-benzimidazol-2-one,
3-(2-aminoethyl)-1-(1-cyclooctylmethyl-4-piperidyl)-1,3-dihydro-2H-benzimidazol-2-one,
1-(1-cyclooctylmethyl-4-piperidyl)-1,3-dihydro-imidazo[4,5-c]pyridin-2-one,
1-[(3RS,4RS)-1-cyclooctylmethyl-3-(2-ethoxycarbonylethyl)-4-piperidyl]-3-ethyl-1,3-dihydro-2H-benzimidazol-2-one,
1-[(3RS,4RS)-1-cyclooctylmethyl-3-(3-hydroxypropyl)-4-piperidyl]-3-ethyl-1,3-dihydro-2H-benzimidazol-2-one,
1-[(3RS,4RS)-1-cyclooctylmethyl-3-ethyl-4-piperidyl]-3-ethyl-1,3-dihydro-2H-benzimidazol-2-one,
1-[(3RS,4RS)-1-(1-ethylcyclooctylmethyl)-3-hydroxymethyl-4-piperidyl]-1,3-dihydro-2H-benzimidazol-2-one,
1-[(3RS,4RS)-1-(1-ethylcyclooctylmethyl)-3-hydroxymethyl-4-piperidyl]-3-propargyl-1,3-dihydro-2H-benzimidazol-2-one,
3-(2-aminoethyl)-1-[(3S*,4S*)-1-cyclooctylmethyl-3-hydroxymethyl-4-piperidyl]-1,3-dihydro-2H-benzimidazol-2-one,
1-[1-(bicyclo[4.4.0]dec-2-ylmethyl)-4-piperidyl]-3-propargyl-1,3-dihydro-2H-benzimidazo)-2-one,
3-benzyl-1-[1-(bicyclo[4.4.0]dec-3-ylmethyl)-4-piperidyl]-1,3-dihydro-2H-benzimidazol-2-one,
3-ethyl-1-[1-(1-ethylcyclooctylmethyl)-4-piperidyl]-1,3-dihydro-2H-benzimidazol-2-one,
1-((3RS,4RS)-1-(1-cyclooctylmethyl)-3-(2-hydroxyethyl)-4-piperidyl]-3-ethyl-1,3-dihydro-2H-benzimidazol-2-one,
3-cyclopropylmethyl-1-[1-(1-ethylcyclooctylmethyl)-4-piperidyl]-1,3-dihydro-2H-benzimidazol-2-one,
1-[1-(1-ethylcyclooctylmethyl)-4-piperidyl]-3-(2-hydroxyethyl)-1,3-dihydro-2H-benzimidazol-2-one,
1-[(3RS,4RS)-1-(1-cyclooctylmethyl)-3-(1,2-dihydroxyethyl)-4-piperidyl]-3-ethyl-1,3-dihydro-2H-benzimidazol-2-one,
3-(2-aminoethyl)-1-[1-(1-ethylcyclooctylmethyl)-4-piperidyl]-1,3-dihydro-2H-benzimidazol-2-one,
1-[(3RS,4RS)-3-carboxyl-1-cyclooctylmethyl-4-piperidyl]-3-ethyl-1,3-dihydro-2H-benzimidazol-2-one,
1-[(3RS,4RS)-3-carbamoyl-1-(1-cyclooctylmethyl)-4-piperidyl]-3-ethyl-1,3-dihydro-2H-benzimidazol-2-one,
1-(1-cyclooctylmethyl)-4-piperidyl-3-[2-(methylamino)ethyl]-1,3-dihydro-2H-benzimidazol-2-one,
1-[(3RS,4RS)-1-(1-cyclooctylmethyl)-3-(2-dimethylaminoethyl)-4-piperidyl]-3-ethyl-1,3-dihydro-2H-benzimidazol-2-one,
1-[(3RS,4RS)-1-(1-cyclooctylmethyl)-3-(3-dimethylaminopropyl)-4-piperidyl]-3-ethyl-1,3-dihydro-2H-benzimidazol-2-one,
1-[(3RS,4RS)-1-(1-cyclooctylmethyl)-3-hydroxymethyl-4-piperidyl]-3-propargyl-1,3-dihydro-2H-benzimidazol-2-one,
1-[(3RS,4RS)-1-(1-cyclooctylmethyl)-3-(1-hydroxyethyl)-4-piperidyl]-3-ethyl-1,3-dihydro-2H-benzimidazol-2-one,
1-[(3RS,4RS)-1-(1-cyclooctylmethyl)-3-(1-hydroxypropyl)-4-piperidyl]-3-ethyl-1,3-dihydro-2H-benzimidazol-2-one,
1-(1-cyclooctylmethyl-4-piperidyl)-3-[(2-(methylsulfonylamino)ethyl]-1,3-dihydro-2H-benzimidazol-2-one,
1-(1-cyclooctylmethyl-4-piperidyl)-3-[(2-(sulfamoylamino)ethyl]-1,3-dihydro-2H-benzimidazol-2-one,
1-(1-cyclooctylmethyl-4-piperidyl)-3-[(2-(dimethylsulfamoylamino)ethyl]-1,3-dihydro-2H-benzimidazol-2-one,
1-[(3RS,4RS)-1-cyclooctylmethyl-3-(3-methylaminopropyl)-4-piperidyl]-3-ethyl-1,3-dihydro-2H-benzimidazol-2-one,
1-[(3S^{*},4S^{*})-1-cyclooctylmethyl-3-(3-methylsulfonylamino)methyl-4-piperidyl]-3-ethyl-1,3-dihydro-2H-benzimidazol-2-one,
1-[(3RS,4RS)-3-(3-aminopropyl)-1-cyclooctylmethyl-4-piperidyl]-3-ethyl-1,3-dihydro-2H-benzimidazol-2-one,
1-[(3RS,4RS)-1-cyclooctylmethyl-3-vinyl-4-piperidyl]-3-ethyl-1,3-dihydro-2H-benzimidazol-2-one,
1-(1-cyclooctylmethyl-3-methylene-4-piperidyl]-3-ethyl-1,3-dihydro-2H-benzimidazol-2-one,
1-[(3RS,4RS)-3-(3-aminoethyl)-1-cyclooctylmethyl-4-piperidyl]-3-ethyl-1,3-dihydro-2H-benzimidazol-2-one,
1-((3S^{*},4S^{*})-1-cyclooctylmethyl-3-(3-dimethylsulfamoyl)amino-4-piperidyl]-3-ethyl-1,3-dihydro-2H-benzimidazol-2-one,
1-[(3S^{*},4S^{*})-1-cyclooctylmethyl-3-(3-sulfamoylamino)methyl-4-piperidyl]-3-ethyl-1,3-dihydro-2H-benzimidazol-2-one,
5-bromo-1-[(3RS,4RS)-1-cyclooctylmethyl-3-hydroxymethyl-4-piperidyl]-3-ethyl-1,3-dihydro-2H-benzimidazol-2-one,
1-[(3RS,4RS)-3-aminoethyl-1-cyclooctylmethyl-4-piperidyl]-3-(2-dimethylaminoethyl)-1,3-dihydro-2H-benzimidazol-2-one,
1-[(3RS,4RS)-1-cyclooctylmethyl-3-(sulfamoylamino)methyl-4-piperidyl]-3-(2-dimethylaminoethyl)-1,3-dihydro-2H-benzimidazol-2-one,
1-[(3RS,4RS)-1-cyclooctylmethyl-3-(methylsulfonylamino)methyl-4-piperidyl]-3-(2-aminoethyl)-1,3-dihydro-2H-benzimidazol-2-one,
1-[(3RS,4RS)-1-cyclooctylmethyl-3-hydroxymethyl-4-piperidyl]-3-(2-fluoroethyl)-1,3-dihydro-2H-benzimidazol-2-one,
1-((3RS,4RS)-1-cyclooctylmethyl-3-hydroxymethyl-4-piperidyl]-3-(2,2-difluoroethyl)-1,3-dihydro-2H-benzimidazol-2-one,
1-[(3RS,4RS)-1-cyclooctylmethyl-3-hydroxymethyl-4-piperidyl]-3-(2,2,2-trifluoroethyl)-1,3-dihydro-2H-benzimidazol-2-one,
1-[1-(5,5-difluorocyclooctylmethyl)-4-piperidyl]-1,3-dihydro-2H-benzimidazol-2-one,
1-(1-cyclooctylmethyl-3-pyrrolidyl)-1,3-dihydro-2H-benzimidazol-2-one,
1-(1-cyclooctylmethyl-3-piperidyl)-1,3-dihydro-2H-benzimidazol-2-one,
3-(2-aminoethyl)-1-[(3RS,4RS)-1-cyclooctylmethyl-3-(sulfamoylamino)methyl-4-piperidyl]-1,3-dihydro-2H-benzimidazol-2-one,
3-carboxymethyl-1-(1-cyclooctylmethyl-4-piperidyl)-1,3-dihydro-2H-benzimidazol-2-one,
1-[(3RS,4RS)-3-amino-1-cyclooctylmethyl-4-piperidyl]-1,3-dihydro-2H-benzimidazol-2-one,
1-(2-cyclooctylmethyl-2-azabicyclo[2.2.2]oct-5-yl)-1,3-dihydro-2H-benzimidazol-2-one,
1-(8-cyclooctylmethyl-8-azabicyclo[4.3.0]non-2-yl)-1,3-dihydro-2H-benzimidazol-2-one,
1-[(3RS,4RS)-1-cyclooctylmethyl-3-(2-pyridylmethyl)oxymethyl-4-piperidyl]-3-ethyl-1,3-dihydro-2H-benzimidazol-2-one,
1-[(3RS,4RS)-1-cyclooctylmethyl-3-(4-pyridylmethyl)oxymethyl-4-piperidyl]-3-ethyl-1,3-dihydro-2H-benzimidazol-2-one,
1-[(3RS,4RS)-1-cyclooctylmethyl-3-(3-pyridylmethyl)oxymethyl-4-piperidyl]-3-ethyl-1,3-dihydro-2H-benzimidazol-2-one,
1-[(3RS,4RS)-3-(carbamoyl amino)methyl-1-cyclooctylmethyl-4-piperidyl]-3-ethyl-1,3-dihydro-2H-benzimidazol-2-one,
1-[(3RS,4RS)-3-carbamoyloxymethyl-1-cyclooctylmethyl-4-piperidyl]-3-ethyl-1,3-dihydro-2H-benzimidazol-2-one,
3-[2-(carbamoyl amino)ethyl]-1-(1-cyclooctylmethyl-4-piperidyl)-1,3-dihydro-2H-benzimidazol-2-one,
1-(1-cyclooctylmethyl-3,3-dimethyl-4-piperidyl)-1,3-dihydro-2H-benzimidazol-2-one,
1-(1-cyclooctylmethyl-r-3,c-5-dimethyl-tert-4-piperidyl)-1,3-dihydro-2H-benzimidazol-2-one,
1-(1-cyclooctylmethyl-r-3,c-5-dimethyl-c-4-piperidyl)-1,3-dihydro-2H-benzimidazol-2-one,
1-[1-(5,5-difluorocyclooctylmethyl)-4-piperidyl]-3-[2-(dimethylamino)ethyl]-1,3-dihydro-2H-benzimidazol-2-one,
3-allyl-1-(1-cyclooctylmethyl-4-piperidyl)-1,3-dihydro-2H-benzimidazol-2-one,
1-(1-cyclooctylmethyl-4-piperidyl)-3-cyclopentyl-1,3-dihydro-2H-benzimidazol-2-one,
1-(1-cyclooctylmethyl-4-piperidyl)-3-hydroxy-1,3-dihydro-2H-benzimidazol-2-one,
1-(1-cyclooctylmethyl-4-piperidyl)-3-methoxy-1,3-dihydro-2H-benzimidazol-2-one,
1-{1-[1-(cyclohexylmethyl)cyclooctyl]methyl-4-piperidyl}-1,3-dihydro-2H-benzimidazol-2-one,
1-[1-(1-benzylcyclooctyl)methyl-4-piperidyl]-1,3-dihydro-2H-benzimidazol-2-one,
1-[1-(tricyclo[3.2.1.1^{3.7}]non-1-ylmethyl)-4-piperidyl]-1,3-dihydro-2H-benzimidazol-2-one,
1-(1-cyclooctylmethyl-4-methoxycarbonyl-4-piperidyl)-1,3-dihydro-2H-benzimidazol-2-one,
3-cyclobutyl-1-(1-cyclooctylmethyl-4-piperidyl)-1,3-dihydro-2H-benzimidazol-2-one,
1-(1-cyclooctylmethyl-4-methoxycarbonyl-4-piperidyl)-3-ethyl-1,3-dihydro-2H-benzimidazol-2-one,
1-(1-cyclooctylmethyl-4-hydroxymethyl-4-piperidyl)-1,3-dihydro-2H-benzimidazol-2-one,
1-(1-cyclooctylmethyl-4-hydroxymethyl-4-piperidyl)-3-ethyl-1,3-dihydro-2H-benzimidazol-2-one,
1-(1-cyclooctylmethyl-4-piperidyl)-3-dimethylamino-1,3-dihydro-2H-benzimidazol-2-one,
1-[(3RS,4RS)-1-cyclooctylmethyl-3-(5-tetrazolylmethyl)-4-piperidyl]-3-ethyl-1,3-dihydro-2H-benzimidazol-2-one,
1-(1-cyclooctylmethyl-4-piperidyl)-3-ethoxycarbonyl-1,3-dihydro-2H-benzimidazol-2-one, and
3-(2-carbamoyloxyethyl)-1-(1-cyclooctylmethyl-4-piperidyl)-1,3-dihydro-2H-benzimidazol-2-one.

As the antipruritic agent, the compounds represented by the formulas (I) to (IV) are preferable, and (1R,2S)-N-amidino-2-{[2-(4-chlorobenzoylamino)-6-methoxyquinazolin-4-yl]amino}cyclohexylamine dihydrochloride, (1R,2S)-N-amidino-2-{[2-(4-chlorobenzoylamino)-6-methylquinazolin-4-yl]amino}cyclohexylamine dihydrochloride, 1-[(3R,4R)-1-cyclooctylmethyl-3-hydroxymethyl-4-piperidyl]-3-ethyl-1,3-dihydro-2H-benzimidazol-2-one hydrochloride and N-(4-amino-2-methyl-6-quinolyl)-2-[(4-ethylphenoxy)methyl]benzamide hydrochloride are particularly preferable.

The compound of the formula (I) of the present invention can be produced, for example, by the method described in International Publication WO 01/72710.

The compound of the formula (II) can be produced, for example, by the methods described in International Publication WO 9307124, Japanese Patent No. 2923742, International Publication WO 9850370, International Publication WO9909986 and Japanese Unexamined Patent Publication No. 47-2927.

The compound of the formula (III) can be produced by the method described in International Publication WO 98/54168

The compound of the formula (IV) can be produced by the method described in International Publication WO 99/48492.

The compounds represented by the formulas (I) to (IV) of the present invention are useful as an antiitching agent and an antipruritic agent because they exert a scratching behavior suppressing effect as shown in the Test Examples described hereinafter.

When the compounds of the present invention are administered as a medicament, they can be administered to a mammal including human as they are or in a mixture with a pharmaceutically acceptable non-toxic inert carrier, for example, as a pharmaceutical composition containing the compound at a level of 0.001% to 99.5%, preferably 0.1% to 90%.

As a carrier, one or more of auxiliary agents for a formulation such as solid, semi-solid and liquid diluent, filler and other auxiliary agents for a drug formulation may be used. It is desirable that a pharmaceutical composition is administered as a unit dosage form. The pharmaceutical composition can be administered into tissue, or orally, intravenously, topically (percutaneously, instillation) or rectally. It is a matter of course that a dosage form suitable for any of the administration modes described above is employed. For example, oral, intravenous or local administration (percutaneous administration, instillation) is preferable.

While it is desirable that the dose as an antipruritic agent may be adjusted depending on the conditions of the patients including the age, body weight, nature and degree of the disease as well as the administration route, a daily dose as an active ingredient in an adult is usually 1 mg to 5 g per adult, preferably 1 mg to 500 mg per adult when given orally, and usually 0.1 mg to 500 mg per adult, preferably 1 mg to 50 mg per adult when given intravenously. The level of the active ingredient is usually 0.01% to 0.5%, preferably 0.01 % to 0.1% when given rectally, and usually 0.001% to 0.5%, preferably 0.001% to 0.01% in case of instillation. In some cases, a lower dose may be sufficient or a higher dose may be required. Usually, the dose is given once or several times as being divided into portions, or given intravenously and continuously over a period of 1 to 24 hours a day.

Oral administration can be accomplished in a solid or liquid dosage form, such as a particle, powder, tablet, sugar-coated tablet, capsule, granule, suspension, liquid, syrup, drop, buccal formulation, suppository or other dosage forms. A particle is produced by pulverizing an active ingredient into a suitable particle size. A powder can be produced by pulverizing an active ingredient into a suitable particle size followed by mixing with a pharmaceutical carrier, such as an edible carbohydrate including starches or mannitol, which has also been pulverized into a suitable particle size. Those which may be added if necessary are flavors, preservatives, dispersing agents, colorants, fragrances and the like.

A capsule may be produced by filling a particle or powder which has previously been pulverized as described above or a granule obtained as described in the section of a tablet for example in a capsule such as a gelatin capsule. It is also possible that an additive such as a lubricant, fluidizing agent, such as colloidal silica, talc, magnesium stearate, calcium stearate or solid polyethylene glycol is mixed with the pulverized material prior to the filling procedure. For the purpose of enhancing the efficacy of a medicament when a capsule is ingested, a disintegrant or solubilizing agent, such as carboxymethyl cellulose, calcium carboxymethyl cellulose, low substituted hydroxypropyl cellulose, sodium croscarmellose, sodium carboxy starch, calcium carbonate or sodium carbonate, may be added.

The finely pulverized powder of the compound of the present invention may be suspended and dispersed in a vegetable oil, polyethylene glycol, glycerin and surfactant, and then encapsulated in a gelatin sheet, thereby obtaining a soft capsule. A tablet is produced by formulating a powder mix, converting into a granule or slug, adding a disintegrant or lubricant and then compacting into a tablet. The powder mix is obtained by mixing an appropriately pulverized material with a diluent or base described above if necessary together with a binder (for example, sodium carboxymethyl cellulose, hydroxypropyl cellulose, methyl cellulose, hydroxypropylmethyl cellulose, gelatin, polyvinyl pyrrolidone, polyvinyl alcohol and the like), a dissolution retardant (for example, paraffin, wax, hardened castor oil and the like), a resorption promoter (for example, quaternary salt), or an adsorbent (for example, bentonite, kaolin, calcium diphosphate and the like). The powder mix can be granulated by wetting with a binder such as a syrup, starch glue, gum arabic, cellulose solution or polymer solution and then forcing to pass through a sieve. Instead of the procedure for granulating a powder as described above, another procedure may be employed in which a mix is subjected first to a tablet compacting machine to form a morphologically incomplete slug which is then ground. A granule thus obtained may contain, as a lubricant, stearic acid, stearates, talc, mineral oil and the like, for the purpose of preventing any adhesion with each other. The mixture thus lubricated is then compacted into tablets. A plane tablet thus obtained may be film-coated or sugar-coated.

An active ingredient may be mixed with a fluidized inert carrier and then compacted directly into tablets without being subjected to the granulating or slugging process described above. A transparent or semi-transparent protective film in the form of a shellac sealing film, a film of a sugar or polymeric material and a glossy film of a wax may also be employed.

Other oral dosage forms, such as a solution, syrup and elixir can be formulated as a unit dosage form whose certain amount contains a certain amount of a medicament. A syrup is produced by dissolving a compound in a flavored aqueous solution, while an elixir is produced by using a non-toxic alcoholic carrier. A suspension is formulated by dispersing a compound in a non-toxic carrier. Additives such as a solubilizing agent, an emulsifier (for example ethoxylated isostearyl alcohols, polyoxyethylene sorbitol esters), a preservative and a flavor (for example, peppermint oil, saccharin) may also be added if necessary.

An oral unit dosage formulation may also be a microcapsule if desired. Such a formulation may be coated or embedded in a polymer or wax to obtain a prolonged activity or sustained release of the active ingredient.

A rectal administration can be accomplished by using a suppository obtained by mixing a compound with a water-soluble or water-insoluble solid having a low melting point such as a polyethylene glycol, cocoa butter, higher esters (for example, myristyl palmitate) as well as a mixture thereof.

The administration into a tissue can be accomplished by using a liquid unit dosage form, for example in the form of a solution or suspension, of a subcutaneous, intramuscular, bladder or intravenous injection formulation. Any of these formulations can be produced by suspending or dissolving a certain amount of a compound in a non-toxic liquid carrier such as an aqueous or oily medium compatible with the purpose of the injection followed by sterilizing said suspension or solution.
Alternatively, a certain amount of a compound is placed in a vial, which is then sterilized together with its content and then sealed. For reconstitution or mixing just before use, a powdery or freeze-dried active ingredient is provided with a complementary vial or carrier. It is also possible to add a non-toxic salt or salt solution for the purpose of making an injection solution isotonic. It is also possible to use in combination with a stabilizer, preservative, emulsifier and the like.

Instillation can be accomplished by using a liquid unit dosage form, for example in the form of a solution or suspension. Any of these formulations can be produced by suspending or dissolving a certain amount of a compound in a non-toxic liquid carrier such as an aqueous or oily medium compatible with the purpose of the instillation followed by sterilizing said suspension or solution. Alternatively, a certain amount of a compound is placed in a vial, which is then sterilized together with its content and then sealed. For reconstitution or mixing just before use, a powdery or freeze-dried active ingredient is provided with a complementary vial or carrier. It is also possible to add a non-toxic salt or salt solution for the purpose of making an ophthalmic solution isotonic. It is also possible to use a stabilizer, preservative, emulsifier and the like.

In the antipruritic agent of the present invention, it is possible to mix or use in combination with other ingredients, for example, an antihistaminic agent, antiallergic agent, steroid and the like.

### BRIEF DESCRIPTION OF DRAWINGS

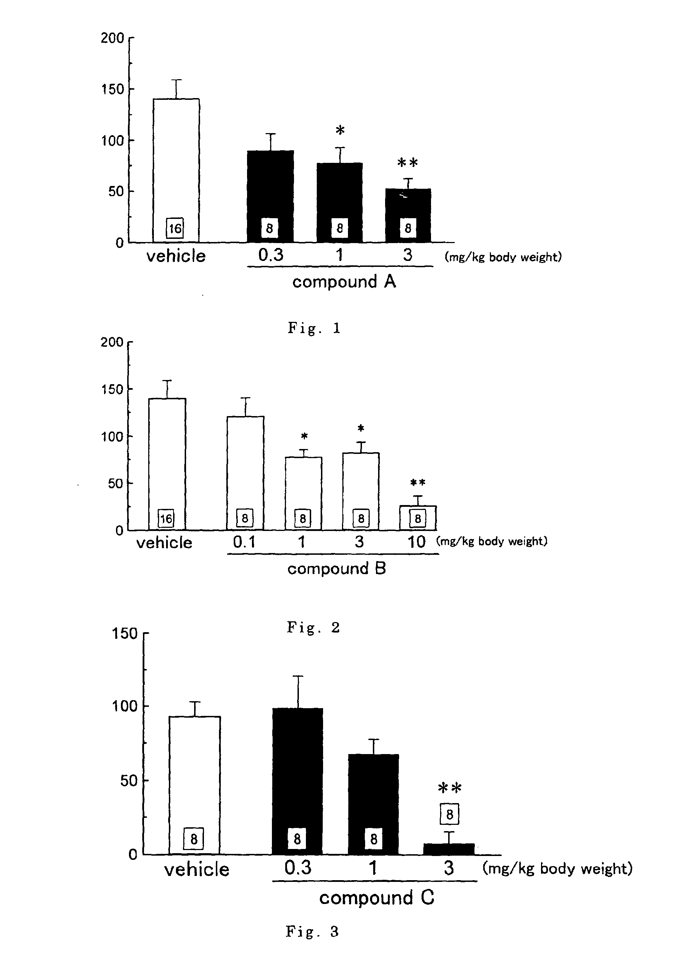

Fig. 1 is a graph showing an influence of intravenous administration of a compound A on a compound 48/80-induced scratching behavior. The ordinate indicates the number of scratching behavior for 30 minutes, which is represented as means ± standard errors, and the numeral in parentheses indicates the number of the respective Examples. The symbol * indicates P < 0.05, and the symbol ** indicates P < 0.01 versus vehicle administration group (Dunnett test).
Fig. 2 is a graph showing an influence of intravenous administration of a compound B on a compound 48/80-induced scratching behavior. The ordinate indicates the number of scratching behavior for 30 minutes, which is represented as means ±standard errors, and the numeral in parentheses indicates the number of the respective Examples. The symbol * indicates P < 0.05, and the symbol ** indicates P < 0.01 versus vehicle administration group (Dunnett test).
Fig. 3 is a graph showing an influence of intravenous administration of a compound C on a compound 48/80-induced scratching behavior. The ordinate indicates the number of scratching behavior for 30 minutes, which is represented as means ± standard errors, and the numeral in parentheses indicates the number of the respective Examples. The symbol ** indicates P < 0.01 versus vehicle administration group (Dunnett test).

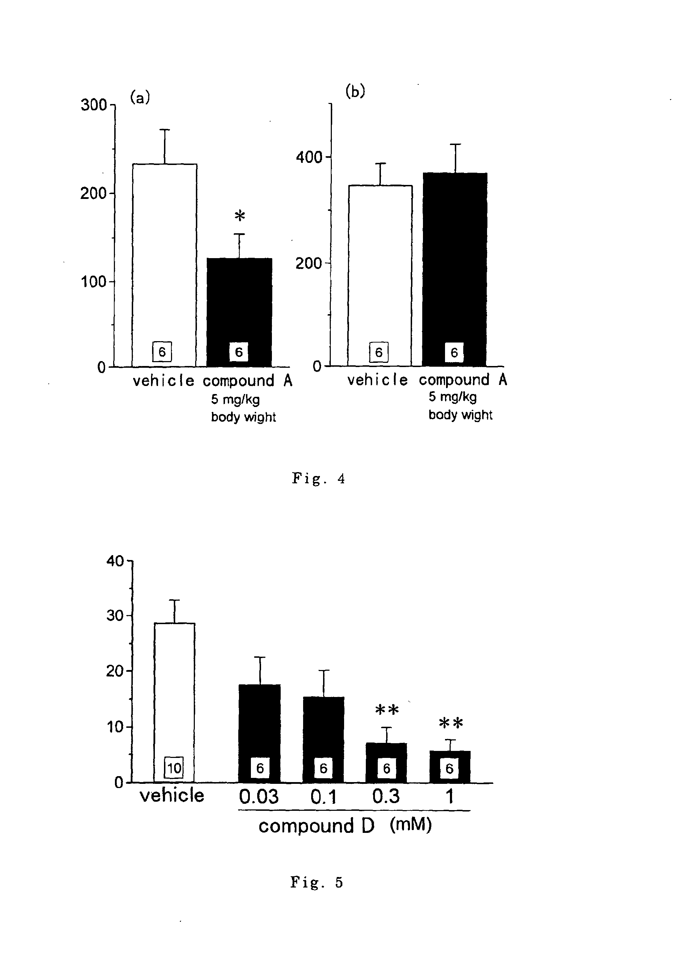

Fig. 4 is a graph showing an influence of intravenous administration of a compound A on a compound 48/80-induced scratching behavior in wild type mice (a) and nociceptin receptor defective mice (b). The ordinate indicates the number of scratching behavior for 30 minutes, which is represented as means ± standard errors, and the numeral in parentheses indicates the number of the respective Examples. The symbol * indicates P < 0.05 versus solvent administration group (t-test).
Fig. 5 is a graph showing an influence of instillation of a compound D on an eye scratching behavior induced by instillation of serotonin. The ordinate indicates the number of scratching behavior for 10 minutes, which is represented as means ± standard errors, and the numeral in parentheses indicates the number of the respective Examples. The symbol * indicates P < 0.05 versus vehicle administration group (t-test).

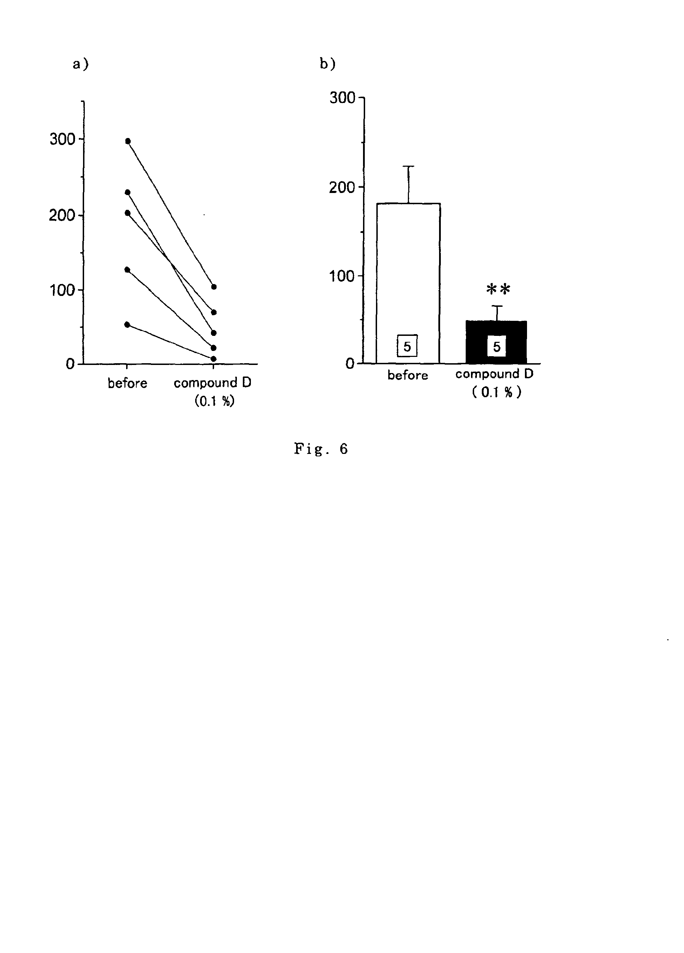

Fig. 6 is a graph showing an influence of application of a compound D on a spontaneous scratching behavior of mice with cutaneous barrier disruption. The ordinate indicates the number of scratching behavior'for 30 minutes. (a) shows the values of individual mice, (b) shows means ± standard errors, and the numeral in parentheses indicates the number of the respective Examples. The symbol ** indicates P < 0.01 versus value before application (paired t-test).

### BEST MODE FOR CARRYING OUT THE INVENTION

The present invention will now be described in more detail with reference to Production Examples of typical starting materials (Reference Examples), Production Examples of the compounds of the present invention (Examples), typical Test Examples and Formulation Examples, but the present invention is not limited thereto. The optical rotation was measured at 20°C. The structure of the compounds of the Examples was confirmed by MS, NMR and elemental analysis.

### Reference Example 1

### Cis-4-tert-butoxycarbonylamino-cis-2-methylcyclohexylamine

### Step 1

### Trans-4-tert-butoxycarbonylamino-trans-2-methylcyclohexanol

To a solution of 1.0 g of trans-4-amino-trans-2-methylcyclohexanol in 20 ml of chloroform, a solution of 2.53 g of di-tert-butyl dicarbonate in 10 ml of chloroform was added dropwise, followed by stirring at room temperature for 15 hours. The reaction solution was concentrated and the residue was purified by silica gel chromatography (n-hexane: ethyl acetate = 2:1) and the resulting crystal was washed with diisopropyl ether to obtain 0.97 g of the desired compound.

### Step 2

### Cis-4-tert-butoxycarbonylamino-cis-2-methyl-N-phthaloylcyclohexylamine

To a solution of 0.97 g of trans-4-tert-butoxycarbonylamino-trans-2-methylcyclohexanol in 50 ml of toluene, 1.35 g of triphenylphosphine was added and 0.75 g of phthalimide and 2.22 g of 40% diethylazodicarboxylate-toluene solution were added dropwise under ice cooling, followed by stirring for 24 hours. The reaction solution was concentrated and the residue was purified by silica gel column chromatography (n-hexane:ethyl acetate = 4:1) to obtain 1.25 of the desired compound.

### Step 3

### Cis-4-tert-butoxycarbonylamino-cis-2-methylcyclohexylamine

To a suspension of 1.25 g of cis-4-tert-butoxycarbonylamino-cis-2-methyl-N-phthaloylcyclohexylamine in 40 ml of ethanol, 1.00 g of hydrazine monohydrate was added, followed by stirring at 80°C for 2 hours. After the solvent was distilled off, the residue was combined with a 10% aqueous solution of sodium hydroxide, and extracted with chloroform. After concentrating while drying over sodium sulfate, the residue was purified by column chromatography on silica gel (chloroform:methanol = 10:1) to obtain 0.75 g of the desired compound.

### Reference Example 2

### (1R,2S,4S)-4-tert-butoxycarbonylamino-2-methylcyclohexylamine

### Step 1

### Trans-4-benzoyloxy-N-tert-butoxycarbonyl-cis-3-methylcyclohexylamine

To a solution of 0.54 g of trans-4-tert-butoxycarbonylamino-trans-2-methylcyclohexanol in 15 ml of methylene chloride, 0.358 g of triethylamine and 0.356 ml of benzoyl chloride were added dropwise under ice cooling, followed by stirring at room temperature for 15 hours. After adding water, the reaction solution was extracted with methylene chloride and then concentrated while drying over magnesium sulfate. The residue was purified by silica gel column chromatography (n-hexane:ethyl acetate = 5:1) to obtain 0.61 g of the desired compound.

### Step 2

### (1S,2S,4S)-4-tert-butoxycarbonylamino-2-methylcyclohexanol

Trans-4-benzoyloxy-N-tert-butoxycarbonyl-cis-3-methylcyclohexylamine was subjected to optical resolution using an optically active column (CHIRALPAK AD column manufactured by DAICEL CHEMICAL INDUSTRIES, L.; n-hexane:isopropyl alcohol:diethylamine = 970:30:1) to obtain a compound of [α]²⁰_{D} + 37.78 (c = 1.0, methanol) obtained from the prior fraction. A benzoyl group of the resulting compound is eliminated in methanol using an aqueous 10% sodium hydroxide solution to obtain the desired compound. Absolute configuration was decided by measuring NMR after the resulting alcohol form was reacted with (+)-α-methoxy-α-(trifluoromethyl)phenylacetyl chloride and (-)-α-methoxy-α-(trifluoromethyl)phenylacetyl chloride to obtain a corresponding ester.

### Step 3

### (1R,2S,4S)-4-tert-butoxycarbonylamino-2-methyl-N-phthaloylcyclohexylamine

In the same manner as in the step 2 of Reference Example 1, the desired compound was obtained from (1R,2S,4S)-4-tert-butoxycarbonylamino-2-methylcyclohexanol.

### Step 4

### (1R,2S,4S)-4-tert-butoxycarbonylamino-2-methylcyclohexylamine

In the same manner as in the step 3 of Reference Example 1, the desired compound was obtained from (1R,2S,4S)-4-tert-butoxycarbonylamino-2-methyl-N-phthaloylcyclohexylamine. Reference Example 3

### (1S,2R,4R)-4-tert-butoxycarbonylamino-2-methylcyclohexylamine

In the same manner as in the steps 2, 3 and 4 of Reference Example 2, the desired compound was obtained from the posterior fraction [α]²⁰_{D} -39.94(c = 1.0,methanol) obtained in the step 2 of Reference Example 2.

### Example 1

### Cis-4-amino-cis-2-methyl-N-{6-methyl-2-[2-(2-pyridyl)ethenyl]quinazolin-4-yl}cyclohexylamine trihydrochloride

### Step 1

### Cis-4-tert-butoxycarbonylamino-cis-2-methyl-N-{6-methyl-2-[2-(2-pyridyl)ethenyl]quinazolin-4-yl}cyclohexylamine

To a solution of 70 mg of 4-chloro-6-methyl-2-[2-(2-pyridyl)ethenyl]quinazoline, 60 mg of cis-4-tert-butoxycarbonylamino-cis-2-methylcyclohexylamine and 100 mg of triethylamine in 10 ml of toluene, a catalytic amount of 4-dimethylaminopyridine was added and the mixture was heated at reflux for 24 hours. After the reaction solution was distilled off, the residue was combined with water, extracted with chloroform and then concentrated while drying over magnesium sulfate. The residue was purified by silica gel column chromatography (chloroform:methanol = 50: 1 ) to obtain 50 mg of the desired compound.

### Step 2

### Cis-4-amino-cis-2-methyl-N-{6-methyl-2-[2-(2-pyridyl)ethenyl]quinazolin-4-yl}cyclohexylamine trihydrochloride

To a solution of 50 mg of cis-4-tert-butoxycarbonylaminocis-2-methyl-N-{6-methyl-2-[2-(2-pyridyl)ethenyl]quinazolin-4-yl}cyclohexylamine in 3 ml of methanol and 3 ml of chloroform, 5 ml of a 4N hydrogen chloride-ethyl acetate solution was added, and the mixture was reacted at 50°C for 48 hours. After concentration, the reaction product was crystallized from ethyl acetate to obtain 40 mg of the desired compound as a pale yellow powder.
Positive ion FAB-MS m/z: 374[M+H)⁺

In the same manner as in Example 1, the following compounds were produced.

### Example 2

### N-[2-(4-chlorostyryl)quinazolin-4-yl]-1,2-ethylenediamine dihydrochloride

Positive ion FAB-MS m/z: 325 [M+H]⁺
Description: white powder

### Example 3

### N-[2-(4-chlorostyryl)quinazolin-4-yl]-1,4-butanediamine dihydrochloride

Positive ion FAB-MS m/z: 353[M+H]⁺
Description: white crystal

### Example 4

### N-[2-(4-chlorostyryl)quinazolin-4-yl]-1,5-pentanediamine dihydrochloride

Positive ion FAB-MS m/z: 367[M+H]⁺
Description: white powder

### Example 5

### N-[2-(4-chlorostyryl)quinazolin-4-yl]-1,6-hexanediamine dihydrochloride

Positive ion FAB-MS m/z: 381 [M+H]⁺
Description: white powder

### Example 6

### N-[6-chloro-2-(4-chlorostyryl)quinazolin-4-yl]-1,6-hexanediamine dihydrochloride

Positive ion FAB-MS m/z: 415[M+H]⁺

### Example 7

### N-[2-(4-chlorostyryl)-6-methylquinazolin-4-yl]-1,6-hexanediamine dihydrochloride

Positive ion FAB-MS m/z: 395[M+H]⁺

### Example 8

### N-[2-(4-chlorostyryl)-6-methylquinazolin-4-yl]-1,4-butanediamine dihydrochloride

Positive ion FAB-MS m/z: 367[M+H]⁺
white powder

### Example 9

### N-(2-(4-chlorostyryl)-6,7-difluoroquinazolin-4-yl]-1,6-hexanediamine dihydrochloride

Positive ion FAB-MS m/z: 417[M+H]⁺
white powder

### Example 10

### N-[2-(4-chlorostyryl)quinazolin-4-yl]-1,8-octanediamine dihydrochloride

Positive ion FAB-MS m/z: 409[M+H]⁺
Description: pale red powder

### Example 11

### Trans-2-[2-(4-chlorostyryl)-6-methylquinazolin-4-yl]aminocyclohexylamine dihydrochloride

Positive ion FAB-MS m/z: 393[M+H]⁺
Description: pale yellow powder

### Example 12

### Cis-2-[2-(4-chlorostyryl)-6-methylquinazolin-4-yl]aminocyclohexylamine dihydrochloride

Positive ion FAB-MS m/z: 393[M+H]⁺
Description: dark pale yellow powder

### Example 13

### N-[2-(4-chlorostyryl)-5-methylquinazolin-4-yl]-1,6-hexanediamine dihydrochloride

Positive ion FAB-MS m/z: 395[M+H]⁺

### Example 14

### N-[2-(4-chlorostyryl)-8-methylquinazolin-4-yl]-1,6-hexanediamine dihydrochloride

Positive ion FAB-MS m/z: 395[M+H]⁺

### Example 15

### N-[2-(4-chlorostyryl)quinazolin-4-yl]-1,4-diamino-2-butene dihydrochloride

Positive ion FAB-MS m/z: 351 [M+H]⁺
Description: pale yellow crystal

### Example 16

### N-[2-(4-chlorostyryl)-6-methylquinazolin-4-yl]-1,2-ethylenediamine dihydrochloride

Positive ion FAB-MS m/z: 339[M+H]⁺
white powder

### Example 17

### N-[2-(4-chlorostyryl)-7-methylquinazolin-4-yl]-1,6-hexanediamine dihydrochloride

Positive ion FAB-MS m/z: 395[M+H]⁺

### Example 18

### N-[2-(4-chlorostyryl)-6-tert-butylquinazolin-4-yl]-1,6-hexanediamine dihydrochloride

Positive ion FAB-MS m/z: 437[M+H]⁺
Description: white powder

### Example 19

### N-[2-(4-chlorostyryl)-6-hydroxyquinazolin-4-yl]-1,6-hexanediamine dihydrochloride

Positive ion FAB-MS m/z: 397[M+H]⁺
orange yellow powder

### Example 20

### N-{2-[2-(3-indolyl)ethenyl]-6-methylquinazolin-4-yl}-1,6-hexanediamine hydrochloride

Positive ion FAB-MS m/z: 400[M+H]⁺

### Example 21

### N-[2-(4-chlorostyryl)-6-methoxyquinazolin-4-yl]-1,6-hexanediamine dihydrochloride

Positive ion FAB-MS m/z: 411[M+H]⁺

### Example 22

### Cis-4-[2-(4-chlorostyryl)-6-methylquinazolin-4-yl]aminocyclohexylamine dihydrochloride

Positive ion FAB-MS m/z: 393[M+H]⁺

### Example 23

### N-[2-(4-chlorostyryl)-6,7-dimethylquinazolin-4-yl]-1,5-pentanediamine dihydrochloride

Positive ion FAB-MS m/z: 395[M+H]⁺

### Example 24

### N-[2-(4-chlorostyryl)-6-isopropylquinazolin-4-yl]-1,6-hexanediamine dihydrochloride

Positive ion FAB-MS m/z: 423[M+H]⁺

### Example 25

### Cis-2-[2-(4-chlorostyryl)-6-methoxyquinazolin-4-yl]aminocyclohexylamine dihydrochloride

Positive ion FAB-MS m/z: 409[M+H]⁺

### Example 26

### Cis-2-[2-(4-chlorostyryl)-6-isopropylquinazolin-4-yl]aminocyclohexylamine dihydrochloride

Positive ion FAB-MS m/z: 421 [M+H]⁺

### Example 27

### Cis-4-[2-(4-chlorostyryl)-6-isopropylquinazolin-4-yl]aminocyclohexylamine dihydrochloride

Positive ion FAB-MS m/z: 421[M+H]⁺

### Example 28

### Cis-4-[2-(4-chlorostyryl)-6-methoxyquinazolin-4-yl]aminocyclohexylamine dihydrochloride

Positive ion FAB-MS m/z: 409[M+H]⁺

### Example 29

### Cis-4-[2-(4-chlorostyryl)-6-ethylquinazolin-4-yl]aminocyclohexylamine dihydrochloride

Positive ion FAB-MS m/z: 407[M+H]⁺

### Example 30

### Cis-3-[2-(4-chlorostyryl)-6-methylquinazolin-4-yl]aminocyclohexylamine dihydrochloride

Positive ion FAB-MS m/z: 393[M+H]⁺

### Example 31

### Trans-3-(2-(4-chlorostyryl)-6-methylquinazolin-4-yl]aminocyclohexylamine dihydrochloride

Positive ion FAB-MS m/z: 393 [M+H)⁺

### Example 32

### Cis-2-[2-(4-chlorostyryl)-6-hydroxyquinazolin-4-yl]aminocyclohexylamine dihydrochloride

Positive ion FAB-MS m/z: 395[M+H]⁺
yellow powder

### Example 33

### Cis-2-[6-benzyloxy-2-(4-chlorostyryl)quinazolin-4-yl]aminocyclohexylamine dihydrochloride

Positive ion FAB-MS m/z: 485[M+H]⁺
pale green powder

### Example 34

### Cis-4-{6-methyl-2-[2-(4-pyridyl)ethenyl]quinazolin-4-yl}aminocyclohexylamine trihydrochloride

Positive ion FAB-MS m/z: 360[M+H]⁺

### Example 35

### Cis-4-[2-(4-chlorostyryl)-6-methoxyquinazolin-4-yl]aminomethylcyclohexylamine dihydrochloride

Positive ion FAB-MS m/z: 423[M+H)⁺

### Example 36

### Trans-4-[2-(4-chlorostyryl)-6-methylquinazolin-4-yl]aminocyclohexylamine dihydrochloride

Positive ion FAB-MS m/z: 393[M+H]⁺

### Example 37

### Cis-4-{6-methyl-2-[2-(3-pyridyl)ethenyl]quinazolin-4-yl}aminocyclohexylamine trihydrochloride

Positive ion FAB-MS m/z: 360[M+H]⁺

### Example 38

### Cis-4-[2-(4-chlorostyryl)-6-ethoxyquinazolin-4-yl]aminocyclohexylamine dihydrochloride

Positive ion FAB-MS m/z: 423[M+H]⁺
orange yellow powder

### Example 39

### Cis-4-aminomethyl-N-[2-(4-chlorostyryl)-6-methoxyquinazolin-4-yl]cyclohexylamine dihydrochloride

Positive ion FAB-MS m/z: 423[M+H]⁺

### Example 40

### Cis-4-{6-methyl-2-[2-(2-pyridyl)ethenyl]quinazolin-4-yl}aminocyclohexylamine trihydrochloride

Positive ion FAB-MS m/z: 360[M+H]⁺

### Example 41

### Cis-4-[2-(4-chlorostyryl)-6-isopropyloxyquinazolin-4-yl]aminocyclohexylamine dihydrochloride

Positive ion FAB-MS m/z: 437[M+H]⁺
yellowish white powder

### Example 42

### Cis-4-amino-N-[2-(4-chlorostyryl)-6-methoxyquinazolin-4-yl]-N-methylcyclohexylamine dihydrochloride

Positive ion FAB-MS m/z: 423[M+H]⁺

### Example 43

### Cis-4-{6-methoxy-2-[2-(3-pyridyl)ethenyl]quinazolin-4-yl}aminocyclohexylamine trihydrochloride

Positive ion FAB-MS m/z: 376[M+H]⁺

### Example 44

### Cis-4-{6-methoxy-2-[2-(2-pyridyl)ethenyl]quinazolin-4-yl}aminocyclohexylamine trihydrochloride

Positive ion FAB-MS m/z: 376[M+H]⁺

### Example 45

### Cis-4-{6-methoxy-2-[2-(4-pyridyl)ethenyl]quinazolin-4-yl}aminocyclohexylamine trihydrochloride

Positive ion FAB-MS m/z: 376[M+H]⁺

### Example 46

### Cis-4-amino-cis-3-methyl-N-[2-(4-chlorostyryl)-6-methoxyquinazolin-4-yl]cyclohexylamine

Positive ion FAB-MS m/z: 423[M+H]⁺
Description: white powder

### Example 47

### Cis-4-[2-(4-chlorostyryl-)-6-hydroxyquinazolin-4-yl]aminocyclohexylamine dihydrochloride

Positive ion FAB-MS m/z: 395[M+H]⁺
pale yellow powder

### Example 48

### Cis-4-amino-cis-2-methyl-N-[2-(4-chlorostyryl)-6-methoxyquinazolin-4-yl]cyclohexylamine

Positive ion FAB-MS m/z: 423[M+H]⁺
Description: white powder

### Example 49

### Cis-4-[2-(2-chlorostyryl)-6-methoxyquinazolin-4-yl]aminocyclohexylamine dihydrochloride

Positive ion FAB-MS m/z: 409[M+H]⁺

### Example 50

### Cis-4-amino-cis-2-methyl-N-{6-methoxy-2-[2-(3-pyridyl)ethenyl]quinazolin-4-yl}cyclohexylamine trihydrochloride

Positive ion FAB-MS m/z: 390[M+H]⁺
Description: pale yellow powder

### Example 51

### Cis-4-amino-cis-2-methyl-N-{6-methoxy-2-[2-(2-pyridyl)ethenyl]quinazolin-4-yl}cyclohexylamine trihydrochloride

Positive ion FAB-MS m/z: 390[M+H]⁺
Description: pale yellow powder

### Example 52

### Cis-4-amino-cis-2-methyl-N-{6-methyl-2-[2-(6-methyl-2-pyridyl)ethenyl]quinazolin-4-yl}cyclohexylamine trihydrochloride

Positive ion FAB-MS m/z: 388[M+H]⁺

### Example 53

### Cis-4-{6-chloro-2-[2-(2-pyridyl)ethenyl]quinazolin-4-yl}aminocyclohexylamine trihydrochloride

Positive ion FAB-MS m/z: 380[M+H]⁺

### Example 54

### 4-{6-methyl-2-[2-(2-pyridyl)ethenyl]quinazolin-4-yl}aminomethylbenzylamine trihydrochloride

Positive ion FAB-MS m/z: 382 [M+H]⁺
Description: yellow powder

### Example 55

### 2-{6-methyl-2-[2-(2-pyridyl)ethenyl]quinazolin-4-yl}aminoethoxyethylamine trihydrochloride

Positive ion FAB-MS m/z: 350 [M+H]⁺
Description: yellow powder

### Example 56

### 2-{6-methoxy-2-[2-(2-pyridyl)ethenyl]quinazolin-4-yl}aminoethylphenylethylamine trihydrochloride

Positive ion FAB-MS m/z: 426 [M+H]⁺
Description: yellow powder

### Example 57

### (1R,2S,4S)-4-amino-2-methyl-N-{6-methyl-2-[2-(2-pyridyl)ethenyl]quinazolin-4-yl}cyclohexylamine trihydrochloride

In the same manner as in Example 1, 60 mg of the desired compound was obtained from 100 mg of 4-chloro-6-methyl-2-[2-(2-pyridyl)ethenyl]quinazoline and 80 mg of (1R,2S,4S)-4-tert-butoxycarbonylamino-2-methylcyclohexylamine.
Positive ion FAB-MS m/z: 374[M+H]⁺
Description: pale yellow powder
Specific rotation: [α]_{D}²⁰ = -34.78/MeOH c = 1.058

### Example 58

### (1S,2R,4R)-4-amino-2-methyl-N-{6-methyl-2-[2-(2-pyridyl)ethenyl]quinazolin-4-yl}cyclohexylamine trihydrochloride

In the same manner as in Example I, 55 mg of the desired compound was obtained from 100 mg of 4-chloro-6-methyl-2-[2-(2-pyridyl)ethenyl]quinazoline and 80 mg of (1S,2R,4R)-4-tert-butoxycarbonylamino-2-methylcyclohexylamine.
Positive ion FAB-MS m/z: 374[M+H]⁺
Description: pale yellow powder
Specific rotation: [α]_{D}²⁰ = +36.82/MeOH c = 0.983

### Example 59

### (1R,2S)-N-amidino-2-{[2-(4-chlorobenzoylamino)-6-methylquinazolin-4-yl]amino}cyclohexylamine dihydrochloride

### Step 1

### (1R,2S)-N-t-butoxycarbonyl-2-(2-chloro-b-methylquinazolin-4-yl)aminocyclohexylamine

To a solution of 4.80 g of 2,4-dichloro-6-methylquinazoline in 100 ml of methylene chloride, 9.12 g of triethylamine and 5.31 g of (1S,2R)-2-(t-butoxycarbonylamino)cyclohexylamine were added, followed by stirring at room temperature for 24 hours. After concentration, the reaction product was combined with water, extracted with methylene chloride and then dried. After the solvent was distilled off, the residue was purified by silica gel column chromatography (chloroform:methanol = 20:1) to obtain 8.30 g of the desired compound.

### Step 2

### (1R,2S)-N-t-butoxycarbonyl-2-[2-(4-methoxybenzylamino)-6-methylquinazolin-4-yl]aminocyclohexylamine

To a solution of 4.00 g of (1R,2S)-N-t-butoxycarbonyl-2-(2-chloro-6-methylquinazolin-4-yl)aminocyclohexylamine and 5.91 g of 4-methoxybenzylamine in 30 ml of N-methyl-2-pyrrolidone, 100 mg of 4-dimethylaminopyridine was added, followed by stirring at 110°C for 24 hours. The reaction solvent was combined with an aqueous 5% acetic acid solution and extracted with ethyl acetate. The organic layer was washed with water and saturated brine and then dried. After the solvent was distilled off, the residue was purified by silica gel column chromatography (chloroform:methanol = 20:1) to obtain 5.10 g of the desired compound.

### Step 3

### (1R,2S)-2-(2-amino-6-methylquinazolin-4-yl)aminocyclohexylamine

To a solution of 9.37 g of (1R,2S)-N-t-butoxycarbonyl-2-{[2-(4-methoxybenzylamino)-6-methylquinazolin-4-yl]amino}cyclohexylamine in 30 ml of methylene chloride, 95 ml of trifluoroacetic acid was added under ice cooling, followed by stirring for 72 hours. The reaction solution was concentrated, neutralized with a saturated sodium hydrogencarbonate solution, extracted with chloroform:methanol = 10:1 and then dried. After the solvent was distilled off, the residue was purified by Fuji Silysia NH silica gel column chromatography (chloroform:methanol = 50:1) to obtain 4.60 g of the desired compound.

### Step 4

### (1R,2S)-N-[N,N'-bis(t-butoxycarbonyl)]amidino-2-[(2-amino-6-methylquinazolin-4-yl)amino]cyclohexylamine

To a solution of 4.53 g of (1R,2S)-2-[(2-amino-6-methylquinazolin-4-yl)amino]cyclohexylamine in 90 ml of methylene chloride, 5.18 g of N,N'-bis(t-butoxycarbonyl)-1H-pyrazole-1-carboxamidine was added, followed by stirring at room temperature for 15 hours. The reaction solution was combined with water, extracted with methylene chloride and then dried. After the solvent was distilled off, the residue was purified by silica gel column chromatography (chloroform:methanol = 20:1) to obtain 8.50 g of the desired compound.

### Step 5

### (1R,2S)-N-[N,N'-bis(t-butoxycarbonyl)]amidino-2-{[2-(4-chlorobenzoylamino)-6-methylquinazolin-4-yl]amino}cyclohexylamine

To a solution of 8.72 ml of N,N-diisopropylethylamine in 40 ml of methylene chloride, 408 mg of 4-dimethylaminopyridine and 4.24 ml of 4-chlorobenzoyl chloride were added, followed by stirring for 30 minutes. To the mixture, a solution of 8.50 g of (1R,2S)-N-[N,N'-bis(t-butoxycarbonyl)]amidino-2-[(2-amino-6-methylquinazolin-4-yl)amino]cyclohexylamine in 50 ml of methylene chloride were added dropwise, followed by stirring at room temperature for one hour. The reaction solution was combined with water, extracted with methylene chloride and then dried. After the solvent was distilled off, the residue was purified by silica gel column chromatography (chloroform:methanol = 30:1) to obtain 9.68 g of the desired compound.

### Step 6

### (1R,2S)-N-amidino-2-{[2-(4-chlorobenzoylamino)-6-methylquinazolin-4-yl]amino}cyclohexylamine dihydrochloride

To a solution of 9.50 g of (1R,2S)-N-[N,N'-bis(t-butoxycarbonyl)]amidino-2-[2-(4-chlorobenzoylamino)-6-methylquinazolin-4-yl]aminocyclohexylamine in 40 ml of methanol and 30 ml of chloroform, 100 ml of a 4N hydrogen chloride-ethyl acetate solution was added and the mixture was reacted at 50°C for 24 hours. After concentration, the reaction product was recrystallized from methanol-ethyl ether to obtain 4.20 g of the desired compound as a colorless powder.
Positive ion FAB-MS m/z: 452[M+H]⁺
Specific rotation [α]²⁰_{D} = -78.61(c = 1.01 methanol)

### Test Example 1

The compound 48/80 (50 µg/0.1 ml) was subcutaneously administered to the back portion of 4-6 weeks-old male ddY mice and the number of scratching behavior in the vicinity of the administered portion were measured for 30 minutes.

As a test compound, (1R,2S)-N-amidino-2-[2-(4-chlorobenzoylamino)-6-methoxyquinazolin-4-yl]aminocyclohexylamine dihydrochloride (compound A), 1-[(3R,4R)-1-cyclooctylmethyl-3-hydroxymethyl-4-piperidyl]-3-ethyl-1,3-dihydro-2H-benzimidazol-2-one hydrochloride (compound B) and N-(4-amino-2-methyl-6-quinolyl)-2-[(4-ethylphenoxy)methyl]benzamide hydrochloride (compound C) were used. Five minutes before the administration of the compound 48/80, the compounds were intravenously administered. In a control group, 0.6% DMSO as a solvent was administered.

The results are shown in Fig. 1 to Fig. 3.

All of the compounds A, B and C exerted a strong scratching behavior suppressing effect. Since all of three nociceptin antagonists having different skeletons exerted an antipruritic effect, this effect is considered to be exerted through a nociceptin receptor.

### Test Example 2

The compound 48/80 (50 µg/0.1 ml) was subcutaneously administered to the back portion of 7-9 weeks-old female wild type and nociceptin receptor defective mice obtained by backcrossing with C57BL/6J and the number of scratching behavior in the vicinity of the administered portion were measured for 30 minutes.

As the test compound, the compound A was used. Five minutes before the administration of the compound 48/80, the compound was intravenously administered. In a control group, distilled water was administered.

The results are shown in Fig. 4.

In the wild type mice, the compound A exerted a strong scratching behavior suppressing effect. The scratching behavior suppressing effect observed in the wild type mice completely disappeared in the nociceptin receptor defective mice. These results indicate that the compound A exerts the scratching behavior suppressing effect through a nociceptin receptor.

### Test Example 3

After the instillation of 10 µl of 1% serotonin hydrochloride (hereinafter referred to as serotonin) in the right eye of 4-6 weeks-old male ICR mice, the number of scratching behavior in the vicinity of the eye induced by the instillation of serotonin for 10 minutes.

As the test compound, (1R,2S)-N-amidino-2-{[2-(4-chlorobenzoylamino)-6-methylquinazolin-4-yl]amino}cyclohexylamine dihydrochloride (compound D) was used. Five minutes before the instillation of serotonin, the instillation of 10 µl of the compound was performed. In a control group, the instillation of distilled water was performed.

The results are shown in Fig. 5.

The compound D strongly suppressed the eye scratching behavior induced by the instillation of serotonin. This result indicates that the nociceptin antagonist is also effectve for itching of eyes when used as an ophthalmic solution.

### Test Example 4

The rostral back portion of 5 weeks-old male ICR mice was shaved under etherization, and then cutaneous barrier was disrupted by carrying out a treatment of applying a mixed solution of acetone and ether (1:1) to the shaved portion and applying distilled water twice a day every day (10 days). Spontaneous scratching behavior in the vicinity of the shaved portion, which is caused by the cutaneous barrier disruption, was monitored by a video under an unmanned condition for 30 minutes before and after administration of the test compound. 100% ethanol was used as the vehicle and the compound D was used as the test compound. The compound was applied (100 µl) in the vicinity of the shaved portion.

The results are shown in Fig. 6.

The compound D (0.1%) strongly suppressed spontaneous scratching behavior induced by the cutaneous barrier disruption. This fact indicates that the nociceptin antagonist is also effective for xeroderma and systemic itching when used as an external agent.

### Formulation Example 1

100 g of the compound A, 292 g of D-mannitol, 120 g of corn starch and 28 g of a low substituted hydroxypropyl cellulose are placed in a fluidized bed granulator (STREA; POWREX) and granulated with spraying a certain amount of an aqueous 5% hydroxypropyl cellulose solution. After drying and then milling by a grinding/milling machine (COMIL; POWREX), a certain amount of magnesium stearate is admixed by a mixer (BOHRE container mixer Model MC20, KOTOBUKIENGINEERING & MANUFACTURING), and the mixture is subjected to a rotary tablet compacting machine (CORRECT 12HUK; KIKUSUI) to mold into tablets each 7 mm in diameter weighing 140 mg per tablet, thereby obtaining a tablet containing 25 mg of the compound of the present invention. Formulation Example 2

75 g of the compound A, 180 g of lactose, 75 g of corn starch and 18 g of carmellose calcium are placed in a stirring granulator (vertical granulator model VG-01), combined with a certain amount of an aqueous 5% hydroxypropylmethyl cellulose solution and granulated, and then dried by a fluidized bed granulating drier (STREA; POWREX) and then milled by a grinding/milling machine (COMIL; manufactured by POWREX). Each 120 mg of the milled material is filled into a #3 capsule using a capsule filling machine (capsule filler; Shionogi Qualicaps), thereby obtaining a capsule containing 25 mg of the compound of the present invention.

### Formulation Example 3

2.5 g of the compound A and 4.5 g of sodium chloride are weighed, combined with 450 mL of water for injection and stirred and dissolved, and adjusted at pH 6.5 with 0.1 mol/L hydrochloric acid or 0.1 mol/L sodium hydroxide. Then water for injection is added to make the entire quantity 500 mL. The solution thus formulated is filtered under pressure through a membrane filter (pore size: 0.22 µm). Then 5.3 mL is filled aseptically to a sterilized 5 mL brown ampoule, thereby obtaining an injection formulation containing 25 mg of the compound of the present invention. The procedure from the preparation through the filling are performed aseptically. Formulation Example 4

99.75 g of WITEPSOL H-15 (manufactured by Hüls) is dissolved at 45°C and combined with 0.25 g of the compound of A, and dispersed by stirring. This was infused into a 1 g suppository mold carefully to prevent sedimentation while hot, solidified and taken out from the mold, thereby obtaining a suppository containing 25 mg of the compound of the present invention. Formulation Example 5

0.5 g of the compound D, 5.2 g of sodium dihydrogenphosphate, 11.9 g of sodium monohydrogenphosphate, 2.5 g of sodium chloride and 0.3 g of benzalkonium chloride were weiged, combined with 950 mL of purified water, and stirred and dissolved. Then purified water is added to make the entire quantity 1000 mL. The solution thus formulated is filtered under pressure through a membrane filter (pore size: 0.2 µm). Then 5 mL is filled aseptically to a sterilized 5 mL eye drop bottle, thereby obtaining an ophthalmic solution (5 mL) containing 0.5 mg/mL of the compound of the present invention. The procedure from the preparation through the filling are performed aseptically.

### Formulation Example 6

80 g of olive oil, 15 g of cetanol and 15 g of stearyl alcohol are weighed, stirred and dissolved while heating to 70°C on a water bath (oil phase). Separately, 1 g of the compound D, 10 g of Polysolvate 80, 5 g of sodium lauryl sulfate, 0.25 g of methyl paraoxybenzoate, 0.15 g of propyl paraoxybenzoate and 880 g of purified water are weighed, stirred and dissolved while heating to 70°C on a water bath (aqueous phase). The oil phase and the aqueous phase are placed in a vacuum emulsifying apparatus and then emulsified while stirring at high speed in a homomixer at 70°C under vacuum. Then, the emulsion is water-cooled to 35°C while stirring at low speed. Then 50 mL is filled to a 50 mL container for lotion, thereby obtaining a lotion (50 mL) containing 1.0 mg/mL of the compound of the present invention.

### Formulation Example 7

250 g of white vaseline, 250 g of stearyl alcohol and 40 g of polyoxyethylene hardened castor oil 60 are weighed, stirred and dissolved while heating to 70°C on a water bath (oil phase). Separately, 1 g of the compound D, 120 g of propylene glycol, 0.25 g of methyl paraoxybenzoate, 0.15 g of propyl paraoxybenzoate and 340 g of purified water are weighed, stirred and dissolved while heating to 70°C on a water bath (aqueous phase). The oil phase and the aqueous phase are placed in a vacuum mixing apparatus and then emulsified while stirring at 70°C under vacuum. An ointment obtained by cooling the emulsion and slowly stirring until the emulsion is solidified is filled to a 10 g ointment bottle or a 10 g ointment tube, thereby obtaining an ointment containing 1.0 mg/g of the compound of the present invention.

### Formulation Example 8

110 g of gelatin, 25 g of polyvinyl alcohol and 10 g of methylcellulose are weighed and mixed to obtain a mixture. The mixture is dispersed in 13 g of glycerin using a small-sized mixer. The mixture is dissolved in 100 g of purified water while heating to 60°C. Furthermore, 85 g of kaolin is added and dispersed at 60°C. A dispersion obtained by mixing 20 g of glycerin with 5 g of sodium poly acrylate is added, and dissolved and dispersed at 60°C. Then 15 g of polybutene is added and dispersed at 60°C. To the dispersion, 0.5 g of the compound D is added and dispersed at 50°C to obtain a paste (containing 0.5 g of the compound D in 500 g). The paste is spread over a support (nonwoven fabric) in a coating weight of 100 g/700 cm², and then the coated support is covered with a liner made of a polyethylene film (50 µm) and cut to obtain a patch. 1 mg of the compound of the present invention is contained in 7 cm² of the patch.

### INDUSTRIAL APPLICABILITY

As is apparent from Fig. 1 to Fig. 6, a nociceptin antagonist has potent scratching behavior suppressing effect, that is, antipruritic effect. This fact indicates that nociceptin antagonists can be used as a preventive or remedy for diseases associated with itching, for example, atopic dermatitis, urticaria, psoriasis, xeroderma, trichophytia and vitiligo vulgaris, local pruritus cutaneous caused by insect excretion and secretion, nodular prurigo, kidney dialysis, diabetes, blood disease, liver disease, kidney disease, incretion and metabolic disorder, viscera malignant tumor, hyperthyroidism, autoimmune disease, multiple sclerosis, neurologic disease, psychoneurosis, allergic conjunctivitis, spring catarrh, atopic keratoconjunctivitis, or itching caused by excess use of laxuries and drugs.

## Claims

1. An antipruritic agent comprising a nociceptin antagonist as an active ingredient.

2. The antipruritic agent according to claim 1, wherein the nociceptin antagonist is a compound which is represented by any one of the following general formulas (I) to (IV), or a salt thereof:
1) the general formula (I): 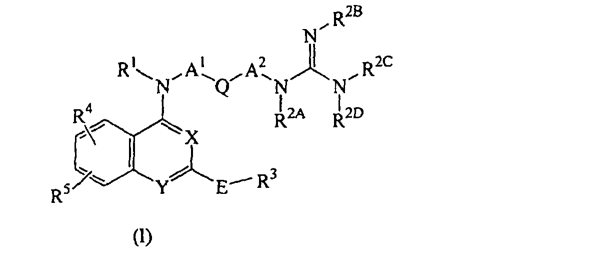 wherein X and Y are the same or different and represent a nitrogen atom or CH;
R¹ represents a hydrogen atom or alkyl;
A¹ and A² are the same or different and represent, (1) a single bond, or (2) a divalent aliphatic hydrocarbon group which may be substituted and may have 1 to 3 unsaturated bonds at any position (the aliphatic hydrocarbon group may contain one hetero atom selected from the group consisting of -NH-, O and S and may include 1 to 3 unsaturated bonds at any position);
Q represents (1) a single bond, (2) an optionally substituted 3- to 8-membered cycloalkylene group, (3) an optionally substituted phenylene group, or (4) an optionally substituted 4-to 8-membered divalent heterocyclic group;
R^{2A}, R^{2C} and R^{2D} are the same or different and represent a hydrogen atom, alkyl or phenyl, and R^{2B} represents a hydrogen atom, alkyl, a cyano group, a nitro group or phenyl, or two nitrogen atoms of a guanidino group are cyclized together with one or two substituents among R^{2B}, R^{2C} and R^{2D} to form a saturated or unsaturated 5- or 6-membered ring;
or are taken together as -N(R¹)-A¹-Q-A²-N(R^{2A})- to form a 5-to 7-membered ring;
E represents (1) an ethenylene group, (2) -NRCO-, (3)-NRCONH-, (4) -CONR-, (5) an ethynylene group, (6) -NRSO₂-, or (7) an aminoalkylene group (wherein R represents hydrogen or an optionally substituted alkyl)
R³ represents an optionally substituted phenyl group or an optionally substituted heterocyclic group;
R⁴ and R⁵ are the same or different and represent (1) a hydrogen atom, alkyl, alkoxy, aralkyloxy, halogen, nitro, hydroxy, alkoxycarbonyl, -NR⁶R⁷, -NR⁶COR⁷, -NR⁶SO₂R⁷, or-CONR⁶R⁷ (wherein R⁶, R⁷ are the same or different and represent a hydrogen atom or alkyl), or (2) adjacent R⁴ and R⁵ may be combined to form -O(CH₂)ₙO- (wherein n represents an integer of 1 or 2) or -CH=CH-CH=CH-;
2) the general formula (II): 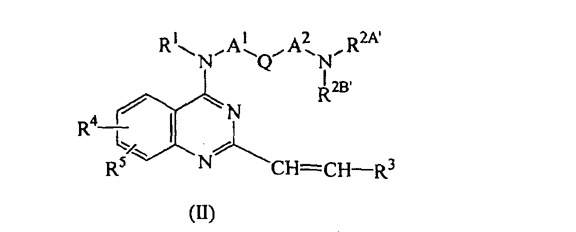 wherein R¹ represents a hydrogen atom or alkyl;
A¹ and A² are the same or different and represent (1) a single bond, or (2) a divalent aliphatic hydrocarbon group which may be substituted and may have 1 to 3 unsaturated bonds at any position (the aliphatic hydrocarbon group may have one hetero atom selected from the group consisting of -NH-, O and S and may include 1 to 3 unsaturated bonds at any position);
Q represents (1) a single bond, (2) an optionally substituted 3- to 8-membered cycloalkylene group, (3) an optionally substituted phenylene group, or (4) an optionally substituted 4-to 8-membered divalent heterocyclic group;
R^{2A'} and R^{2B'} are the same or different and represent a hydrogen atom or alkyl;
or are taken together as -N(R¹)-A¹-Q-A²-N(R^{2A'})- to form a 5-to 7-membered ring;
R³ represents an optionally substituted phenyl group or an optionally substituted heterocyclic group;
R⁴ and R⁵ are the same or different and represent, (1) a hydrogen atom, alkyl, alkoxy, aralkyloxy, halogen, nitro, hydroxy, alkoxycarbonyl, -NR⁶R⁷, -NR⁶COR⁷, -NR⁶SO₂R⁷, or-CONR⁶R⁷ (wherein R⁶ and R⁷ are the same or different and represent a hydrogen atom or alkyl), or (2) adjacent R⁴ and R⁵ may be combined to form -O(CH₂)ₙO- (wherein n represents an integer of 1 or 2) or -CH=CH-CH=CH-);
3) a compound represented by the general formula (III): 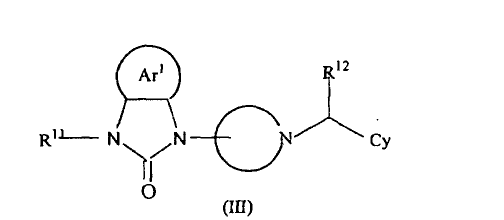 a compound which is represented as followed, or a salt thereof or a ester thereof; 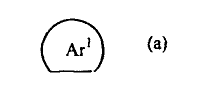 [wherein represents an aromatic carbon ring or an aromatic heterocycles, which may have a substituent selected from the group consisting of halogen atom, lower alkyl group, amino group, lower alkylamino group, di-lower alkylamino group, hydroxyl group, lower alkoxy group and carboxyl group; Cy represents a C3-20 mono-, di- or tricyclic aliphatic carbon ring group which may have a substituent selected from the group consisting of halogen atom, lower alkylidene group, lower alkenyl group, lower alkynyl group, amino group, lower alkylamino group, di-lower alkylamino group, lower alkoxy group and group represented by -R¹⁴; 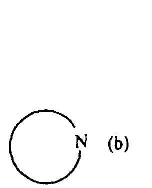 represents a C3-14 mono- or dicyclic aliphatic nitrogen-containing heterocyclic group which may have a substituent selected from the group consisting of halogen atom, lower alkylidene group, lower alkenyl group, lower alkynyl group, amino group, lower alkylamino group, di-lower alkylamino group, hydroxyl group, lower alkoxy group, carboxyl group, lower alkoxycarbonyl group, carbamoyl group, lower alkylcarbamoyl group, di-lower alkylcarbamoyl group and group represented by -R¹³; R¹¹ represents a hydrogen atom, a lower alkenyl group, a lower alkynyl group, a lower cycloalkyl group, an amino group, a lower alkylamino group, a di-lower alkylamino group, a hydroxyl group, a lower alkoxy group, a carboxyl group, a lower alkoxycarbonyl group, a carbamoyl group, a lower alkylcarbamoyl group or a di-lower alkylcarbamoyl group, or a lower alkyl group which may have a substituent selected from the group consiting of halogen atom, lower cycloalkyl group, amino group, lower alkylamino group, di-lower alkylamino group, lower alkylsulfonylamino group, aminosulfonylamino group, (lower alkylamino)sulfonylamino group, (di-lower alkylamino)sulfonylamino group, carbamoyl amino group, (lower alkylcarbamoyl)amino group, (di-lower alkylcarbamoyl)amino group, hydroxyl group, lower alkoxy group, carbamoyloxy group, lower alkylcarbamoyloxy group, di-lower alkylcarbamoyloxy group, carboxyl group, lower alkoxycarbonyl group, carbamoyl group, lower alkylcarbamoyl group, di-lower alkylcarbamoyl group and group represented by -Ar²; R¹² represents a hydrogen atom or a lower alkyl group; R¹³ represents a lower alkyl group which may have a substituent selected from the group consisting of amino group, lower alkylsulfonylamino group, aminosulfonylamino group, (lower alkylamino)sulfonylamino group, (di-lower alkylamino)sulfonylamino group, carbamoyl amino group, (lower alkylcarbamoyl)amino group, (di-lower alkylcarbamoyl)amino group, hydroxyl group, carbamoyloxy group, lower alkylcarbamoyloxy group, di-lower alkylcarbamoyloxy group, carboxyl group, lower alkoxycarbonyl group, carbamoyl group, lower alkylcarbamoyl group, di-lower alkylcarbamoyl group, aromatic heterocycle and group represented by -R¹⁵; R¹⁴ represents a lower alkyl group which may have a substituent selected from the group consisting of C3-10 cycloalkyl group, aromatic carbon ring group and aromatic heterocyclic group; R¹⁵ represents a lower alkylamino group, a di-lower alkylamino group or a lower alkoxy group, which may have an aromatic carbon ring group or an aromatic heterocyclic group; and Ar² represents an aromatic carbon ring group or an aromatic heterocyclic group, which may have a substituent selected from the group consisting of halogen atom, lower alkyl group, amino group, lower alkylamino group, di-lower alkylamino group, hydroxyl group, lower alkoxy group and carboxyl group]; and
4) a compound represented by the general formula (IV): 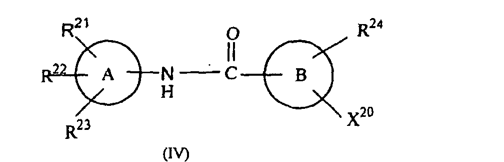 an amide derivative which is represented as followed, or a pharmaceutically acceptable salt thereof;
[wherein R²¹ and R²² are the same or different and each represents a hydrogen atom, a lower alkyl group which may be substituted with a hydroxyl group, an amino group, a lower alkylamino group or a di-lower alkylamino group; R²³ and R²⁴ are the same or different and each represents a hydrogen atom, a halogen atom or a lower alkyl group, the ring A represents an aryl group or a heterocyclic group, the ring B represents a phenyl group, a thienyl group, a furyl group, a pyrrolyl group, apyrrolidinyl group, an oxazolyl group or a cyclohexenyl group, X²⁰ represents a hydrogen atom, a halogen atom, a lower alkyl group which may be substituted with a lower alkoxy group, a lower alkenyl group, an amino group, a cyano group, or 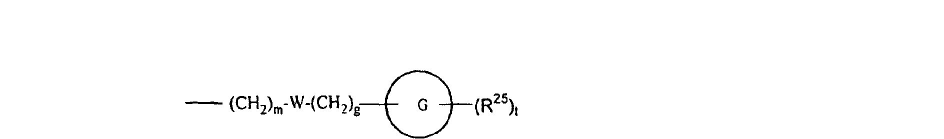 {wherein W represents a single bond, -CH=CR²⁶- (wherein R²⁶ represents a hydrogen atom or an aryl group), -0-, -S-, -NR²⁷- (wherein R²⁷ represents a hydrogen atom, a lower alkyl group or a lower alkoxycarbonyl group), a carbonyl group, a sulfinyl group or -NHCO-, the ring G represents an aryl group, a heterocyclic group, a cycloalkyl group or a fused aryl group, R²⁵ represents a halogen atom, a hydroxyl group, a lower alkoxy group which may be substitiuted with a lower alkoxy group, a lower alkyl group which may be substituted with any of a halogen atom, a hydroxyl group and a lower alkanoyloxy group, a lower alkoxy group which may be substituted with a lower alkoxy group, an amino group, a lower alkylamino group, a di-lower alkylamino group, a nitro group, a cyano group, a lower alkanoyl group, a lower alkanoyloxy group, a carboxy group, a lower alkoxycarbonyl group, a lower alkylsulfonyl group or a phenyl group, t is an integer of 0 or 1 to 5, which represents the number of substituents on the ring G, and when t is an integer of 2 to 5, R²⁵ may be the same or different, m represents an integer of 0 or 1 to 8, and g represents an integer of 0 or 1 to 4.}]

3. The antipruritic agent according to claim 1, wherein the nociceptin antagonist is a compound represented by the formula (I), (III) or (IV) of claim 2.

4. The antipruritic agent according to claim 1, wherein the nociceptin antagonist is a compound selected from the group consisting of (1R,2S)-N-amidino-2-{[2-(4-chlorobenzoylamino)-6-methoxyquinazolin-4-yl] amino}cyclohexylamine dihydrochloride, (1R,2S)-N-amidino-2-{[2-(4-chlorobenzoylamino)-6-methylquinazolin-4-yl]amino}cyclohexylamine dihydrochloride, 1-[(3R,4R)-1-cyclooctylmethyl-3-hydroxymethyl-4-piperidyl]-3-ethyl-1,3-dihydro-2H-benzimidazol-2-one hydrochloride and N-(4-amino-2-methyl-6-quinolyl)-2-[(4-ethylphenoxy)methyl]benzamide hydrochloride.

5. An antipruritic agent comprising a therapeutically active amount of a nociceptin antagonist and a pharmaceutically acceptable carrier or excipient.
